# EUROPEAN PATENT APPLICATION

(11) **EP 4 706 506 A1**
(43) Date of publication of application: **11.03.2026**
(21) Application number: 24938234.2
(22) Date of filing: 15.05.2024
(51) Int. Cl.: A61B 5/00

(54) **PHYSIOLOGICAL SIGNAL MONITORING APPARATUS**

(71) Applicant: Ascend Technology Limited, Hong Kong 999077 (HK)
(72) Inventor: ZHOU, Xin, Shenzhen, Guangdong 518108 (CN); SU, Qi, Shenzhen, Guangdong 518108 (CN); ZHANG, Yuxiang, Shenzhen, Guangdong 518108 (CN); NING, Yixuan, Shenzhen, Guangdong 518108 (CN); LI, Bolun, Shenzhen, Guangdong 518108 (CN); LIAO, Fengyun, Shenzhen, Guangdong 518108 (CN); QI, Xin, Shenzhen, Guangdong 518108 (CN)
(74) Representative: Wang, Bo
(86) International application number: PCT/CN2024/093342
(87) International publication number: WO 2025/236202

(57) **Abstract**

Provided is a device for monitoring a physiological signal. The device for monitoring the physiological signal includes: a strap configured to be worn on a body of a user, the strap includes an inner surface and an outer surface; a plurality of electrodes arranged on the inner surface of the strap and configured to receive electrical signals from the body of user, at least a portion of the electrode on the strap 331 is located between a first portion of one or more conductive threads and the body of user, and a second portion of the one or more conductive thread extends to the inner surface and electrically connects the first portion of the one or more conductive threads to the body of the user, providing electrostatic shielding for the plurality of electrodes.

## Description

### TECHNICAL FIELD

The present disclosure relates to a field of signal monitoring, and in particular to a device for monitoring a physiological signal.

### BACKGROUND

A heart rate strap is a common device for monitoring a physiological signal. The heart rate strap may include two electrocardiogram electrodes. An electrocardiographic signal is obtained from a potential difference between potentials collected at the respective positions of the two electrocardiogram electrodes. However, static electricity generated from a contact between the heart rate strap and clothing affects the potentials collected by the two electrocardiogram electrodes, thereby affecting the accuracy of the obtained electrocardiographic signal. For example, an excessive electrostatic charge will result in an inadequately low signal-to-noise ratio in the electrocardiographic signal.

Therefore, it is necessary to provide a device for monitoring a physiological signal, which can achieve electrostatic shielding for the electrocardiogram electrodes and improve the signal-to-noise ratio of the electrocardiographic signal.

### SUMMARY

One or more embodiments of the present disclosure provide a device for monitoring a physiological signal, the device includes: a strap configured to be worn on a body of a user, the strap including an inner surface and an outer surface; and a plurality of electrodes arranged on the inner surface of the strap and configured to receive electrical signals from the body of the user, the one or more conductive threads are arranged on the strap, a first portion of the one or more conductive threads is located on the outer surface or between the inner surface and the outer surface, such that each of the plurality of electrodes is at least partially located between the first portion of the one or more conductive threads and the body of the user, and a second portion of the one or more conductive threads extends to the inner surface and electrically connects the first portion of the one or more conductive threads to the body of the user to provide electrostatic shielding for the plurality of electrodes.

In some embodiments, the strap is at least formed by weaving elastic threads, and the one or more conductive threads are interwoven through the inner surface and the outer surface of the strap.

In some embodiments, the strap is at least formed by a mixed weaving of elastic threads and the one or more conductive threads.

In some embodiments, a portion of the strap is formed by weaving the one or more conductive threads, and the one or more conductive threads penetrate through the inner surface and the outer surface in a thickness direction of the strap.

In some embodiments, the first portion and the second portion of the one or more conductive threads include a plurality of conductive channels arranged side by side in a width direction of the strap, respectively.

In some embodiments, each of the plurality of conductive channels is formed by weaving the one or more conductive threads in a wave pattern along a length direction of the strap.

In some embodiments, the plurality of electrodes include a first electrode and a second electrode spaced apart along a length direction of the strap, the first portion of the one or more conductive threads forms a first conductive region and a second conductive region insulated from each other, the first conductive region covers an outer side of the first electrode and is insulated from the first electrode, and the second conductive region covers an outer side of the second electrode and is insulated from the second electrode.

In some embodiments, the first conductive region covers the first electrode in the length direction of the strap, and the second conductive region covers the second electrode in the length direction of the strap.

In some embodiments, the first conductive region covers the first electrode in a width direction of the strap, and the second conductive region covers the second electrode in the width direction of the strap.

In some embodiments, the plurality of electrodes include a first electrode and a second electrode spaced apart along a length direction of the strap, the first portion of the one or more conductive threads forms a conductive region, and the conductive region covers the first electrode and the second electrode in the length direction.

In some embodiments, the conductive region covers the first electrode and the second electrode in a width direction of the strap.

In some embodiments, the plurality of electrodes are attached to the strap via one or more insulating layers, and the one or more insulating layers provide insulation between the plurality of electrodes and the one or more conductive threads.

In some embodiments, the one or more conductive threads include metal fibers, carbon fibers, compound fibers, or polymer fibers.

One or more embodiments of the present disclosure provide a device for monitoring a physiological signal, the device for monitoring a physiological signal includes: a strap configured to be worn on a body of the user, the strap including an inner surface and an outer surface; a plurality of signal electrodes arranged on the inner surface of the strap and configured to receive electrical signals from the body of the user; two sets of electrostatic protection sheets, the one set of the two sets of electrostatic protection sheets is disposed on a first region of the outer surface of the strap or is disposed between the inner surface and the first region of the outer surface, the other set of the two sets of electrostatic protection sheets is disposed on a second region of the outer surface of the strap or is disposed between the inner surface and the second region of the outer surface, such that the plurality of signal electrodes are located between the two sets of electrostatic protection sheets and the body of the user, the each set of electrostatic protection sheets includes a plurality of electrostatic protection sheets; and a ground electrode located on the inner surface of the strap and configured to electrically connect the two sets of electrostatic protection sheets to the body of the user to provide electrostatic shielding for the plurality of signal electrodes.

In some embodiments, at least a portion of the plurality of electrostatic protection sheets are electrically connected by one or more conductive threads.

In some embodiments, a distance between connection points of two adjacent electrostatic protection sheets and one of the one or more conductive threads connecting the two adjacent electrostatic protection sheets is less than a natural length of the one of the one or more conductive threads.

In some embodiments, the one or more conductive threads are elastic conductive threads, and the one or more conductive threads are elastically stretchable along an axial direction of the one or more conductive threads.

In some embodiments, the plurality of signal electrodes include a first signal electrode and a second signal electrode spaced apart along a length direction of the strap, the plurality of electrostatic protection sheets are electrically connected in sequence by the one or more conductive threads, and the plurality of electrostatic protection sheets form a conductive region that covers the first signal electrode and the second signal electrode in the length direction of the strap.

In some embodiments, the conductive region covers the first signal electrode and the second signal electrode in a width direction of the strap.

In some embodiments, the plurality of signal electrodes include a first signal electrode and a second signal electrode spaced apart along a length direction of the strap, a portion of the plurality of electrostatic protection sheets are electrically connected by a part of the one or more conductive threads to form a first conductive region that covers an outer side of the first signal electrode, another portion of the plurality of electrostatic protection sheets are electrically connected by another part of the one or more conductive threads to form a second conductive region that covers an outer side of the second signal electrode, and the first conductive region and the second conductive region are insulated from each other.

In some embodiments, the first conductive region covers the first signal electrode in the length direction of the strap, and the second conductive region covers the second signal electrode in the length direction of the strap.

In some embodiments, the first conductive region covers the first signal electrode in a width direction of the strap, and the second conductive region covers the second signal electrode in the width direction of the strap.

In some embodiments, the plurality of electrostatic protection sheets are arranged side by side along a length direction of the strap.

In some embodiments, the electrostatic protection sheets in each set are connected end-to-end in a stacked manner to form a bending structure, and the bending structure is elastically stretchable along a length direction of the strap.

### BRIEF DESCRIPTION OF THE DRAWINGS

The present disclosure will be further illustrated by way of exemplary embodiments, which will be described in details by means of the accompanying drawings. These embodiments are not limiting, and in these embodiments, the same counting denotes the same structure, wherein:
FIG. 1 is a schematic diagram illustrating an application scenario of a device for monitoring a physiological signal according to some embodiments of the present disclosure;
FIG. 2A is a diagram illustrating a structure of an inner surface of a traditional device for monitoring a physiological signal according to some embodiments of the present disclosure;
FIG. 2B is a diagram illustrating a structure of an outer surface of a traditional device for monitoring a physiological signal according to some embodiments of the present disclosure;
FIG. 3A is a diagram illustrating a structure of an inner surface of a device for monitoring a physiological signal according to some embodiments of the present disclosure;
FIG. 3B is a diagram illustrating a structure of an outer surface of a device for monitoring a physiological signal according to some embodiments of the present disclosure;
FIG. 3C is a diagram illustrating a front view of a device for monitoring a physiological signal according to some embodiments of the present disclosure;
FIG. 3D is a diagram illustrating another front view of a device for monitoring a physiological signal according to some embodiments of the present disclosure;
FIG. 3E is a diagram illustrating another structure of an inner surface of a device for monitoring a physiological signal according to some embodiments of the present disclosure;
FIG. 3F is a diagram illustrating another structure of an outer surface of a device for monitoring a physiological signal according to some embodiments of the present disclosure;
FIG. 3G is a schematic diagram illustrating a front projection view of a device for monitoring a physiological signal according to some embodiments of the present disclosure;
FIG. 3H is a schematic diagram illustrating a side projection view of a device for monitoring a physiological signal according to some embodiments of the present disclosure;
FIG. 3I is a schematic diagram illustrating another front projection view of a device for monitoring a physiological signal according to some embodiments of the present disclosure;
FIG. 3J is a schematic diagram illustrating another side projection view of a device for monitoring a physiological signal according to some embodiments of the present disclosure;
FIG. 4A is a diagram illustrating a structure of an inner surface of another device for monitoring a physiological signal according to some embodiments of the present disclosure;
FIG. 4B is a diagram illustrating a structure of an outer surface of another device for monitoring a physiological signal according to some embodiments of the present disclosure;
FIG. 4C is a diagram illustrating another structure of an outer surface of another device for monitoring a physiological signal according to some embodiments of the present disclosure;
FIG. 4D is a schematic diagram illustrating a front projection view of another device for monitoring a physiological signal according to some embodiments of the present disclosure;
FIG. 4E is a schematic diagram illustrating a side projection view of another device for monitoring a physiological signal according to some embodiments of the present disclosure;
FIG. 4F is a schematic diagram illustrating another front projection view of another device for monitoring a physiological signal according to some embodiments of the present disclosure;
FIG. 4G is a schematic diagram illustrating another side projection view of another device for monitoring a physiological signal according to some embodiments of the present disclosure;
FIG. 4H is an exemplary diagram illustrating a connection method of electrostatic protection sheets according to some embodiments of the present disclosure;
FIG. 5A is a diagram illustrating a verification result of a device for monitoring a physiological signal without an anti-static design in a high-impedance state according to some embodiments of the present disclosure;
FIG. 5B is a diagram illustrating a verification result of a device for monitoring a physiological signal with an anti-static design in a high-impedance state according to some embodiments of the present disclosure;
FIG. 5C is a diagram illustrating a verification result of a device for monitoring a physiological signal without an anti-static design in a low-impedance state according to some embodiments of the present disclosure; and
FIG. 5D is a diagram illustrating a verification result of a device for monitoring a physiological signal with an anti-static design in a low-impedance state according to some embodiments of the present disclosure.

### DETAILED DESCRIPTION

In order to provide a clearer understanding of the technical solutions of the embodiments described in the present disclosure, a brief introduction to the drawings required in the description of the embodiments is given below. It is evident that the drawings described below are merely some examples or embodiments of the present disclosure, and for those skilled in the art, the present disclosure may be applied to other similar situations without exercising creative labor. Unless otherwise indicated or stated in the context, the same reference numerals in the drawings represent the same structures or operations.

It should be understood that the terms "system", "device", "unit", and/or "module" used herein are ways for distinguishing different components, elements, parts, sections, or assemblies at different levels. However, if other terms can achieve the same purpose, they may be used as alternatives.

As indicated in the present disclosure and in the claims, the singular forms "a", "an", and "the" may be intended to include the plural forms as well, unless the context clearly indicates otherwise. Generally, the terms "include" and "comprise" only suggest the inclusion of explicitly identified steps and elements. These steps and elements do not constitute an exclusive list. A method or device may also contain other steps or elements.

Flowcharts are used in the present disclosure to illustrate the operations performed by the system according to the embodiments described herein. It should be understood that the operations may not necessarily be performed in the exact sequence depicted. Instead, the operations may be performed in reverse order or concurrently. Meanwhile, other operations may be added to these processes, or one or more operations may be removed.

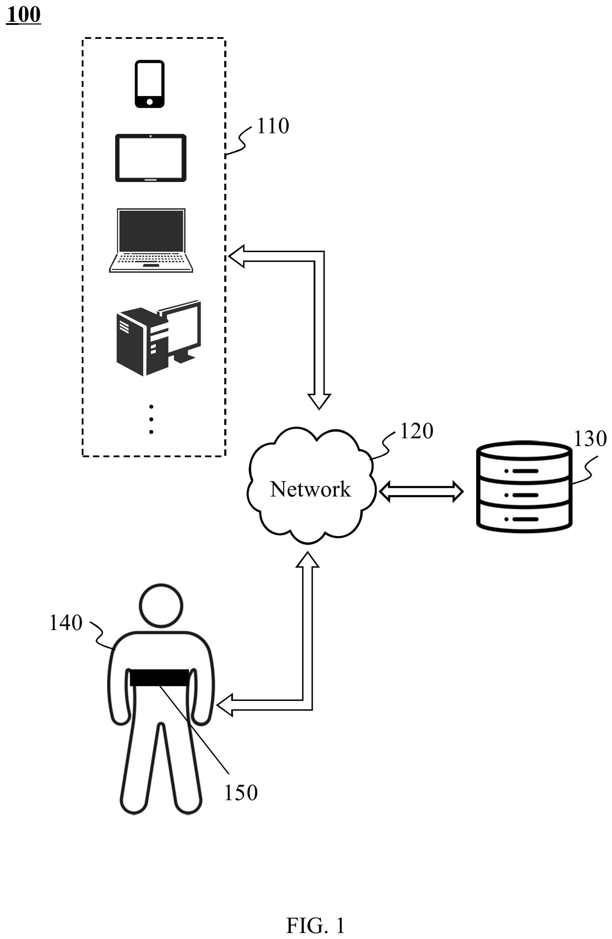

FIG. 1 is a schematic diagram illustrating an application scenario of a device for monitoring a physiological signal according to some embodiments of the present disclosure. As shown in FIG. 1, in some embodiments, an application scenario 100 of a device for monitoring a physiological signal (hereinafter referred to as the application scenario 100) may include a terminal device 110, a network 120, a storage device 130, a monitoring object 140, and a device 150 for monitoring a physiological signal. In some embodiments, various components in the application scenario 100 (e.g., the terminal device 110, the storage device 130, and the device 150 for monitoring the physiological signal) may be connected to and/or communicate with each other via the network 120 (e.g., a wireless connection, a wired connection, or a combination thereof).

The terminal device 110 refers to a device and/or software used by a user related to the application scenario 100. The user related to the application scenario 100 includes, but is not limited to, the monitoring object 140, a physician (e.g., a clinician or a radiotherapist), a nurse, etc. For example, the terminal device 110 may be a device or software configured to control the device 150 for monitoring the physiological signal. The user issues a control instruction to the device 150 for monitoring the physiological signal via the terminal device 110, thereby controlling the device 150 for monitoring the physiological signal to collect a physiological signal of the monitoring object 140. For example, the terminal device 110 may send the control instruction input by the user to the device 150 for monitoring the physiological signal via the network 120, thereby controlling the device 150 for monitoring the physiological signal to collect the physiological signal of the monitoring object 140. In some embodiments, the terminal device 110 may obtain the physiological signal of the monitoring object 140 collected by the device 150 for monitoring the physiological signal via the network 120. In some embodiments, the terminal device 110 may be a mobile device, a tablet computer, a laptop computer, a desktop computer, or other devices with input and/or output functions, or any combination thereof.

The physiological signal refers to a bioelectrical signal (e.g., an electrocardiographic (ECG) signal, an electromyography (EMG) signal) of an object (e.g., the monitoring object 140) collected by a signal collection device (e.g., the device 150 for monitoring the physiological signal). The physiological signal may be a mixed signal containing a noise signal (e.g., a motion artifact signal or an electrostatic signal) and a pure physiological signal. The pure physiological signal refers to a true physiological signal of the object (e.g., the monitoring object 140) obtained after filtering out the noise signal.

The network 120 may connect various components (e.g., the terminal device 110, the storage device 130, the device 150 for monitoring the physiological signal) of the application scenario 100 and/or connect the application scenario 100 with external resources. The network 120 may realize a communication between various components of the application scenario 100 and between these components and other parts outside the application scenario 100, thereby facilitating the exchange of data and/or information. For example, the terminal device 110 may obtain the physiological signal of the monitoring object 140 collected by the device 150 for monitoring the physiological signal via the network 120. As another example, the storage device 130 may obtain and store physiological signal data of the monitoring object 140 collected by the device 150 for monitoring the physiological signal via the network 120.

In some embodiments, the network 120 may be any form of a wired network, a wireless network, or any combination thereof. Merely by way of example, the network 120 may include a cable network, the wired network, a fiber optic network, a telecommunication network, an intranet, the internet, a local area network (LAN), a wide area network (WAN), a wireless local area network (WLAN), a metropolitan area network (MAN), a public switched telephone network (PSTN), a Bluetooth network, a ZigBee network, a near field communication (NFC) network, etc., or any combination thereof. In some embodiments, the network 120 may include at least one network access point. At least one component of the application scenario 100 may connect to the network 120 via the access point to exchange data and/or information. For example, the collected physiological signal and other data may be transmitted via the network 120.

The storage device 130 may store data, instructions, and/or any other information. In some embodiments, the storage device 130 may store data obtained from the device 150 for monitoring the physiological signal and/or the terminal device 110. For example, the storage device 130 may store the physiological signal collected by the device 150 for monitoring the physiological signal. In some embodiments, the storage device 130 may include a mass storage, a removable storage, a volatile read-write memory, a read-only memory (ROM), etc., or any combination thereof. Exemplary mass storages may include a magnetic disk, an optical disk, a solid-state disk, etc. In some embodiments, the storage device 130 may be implemented on a cloud platform. Merely by way of example, the cloud platform may include a private cloud, a public cloud, a hybrid cloud, a community cloud, a distributed cloud, an internal cloud, a multilayer cloud, etc., or any combination thereof.

In some embodiments, the storage device 130 may be connected to the network 120 to communicate with at least one of other components in the application scenario 100. The at least one component in the application scenario 100 may access the data, the instructions, or other information stored in the storage device 130 via the network 120. In some embodiments, the storage device 130 may be directly connected to or communicate with one or more components (e.g., the device 150 for monitoring the physiological signal, or the terminal device 110) in the application scenario 100. In some embodiments, the storage device 130 may be part of the device 150 for monitoring the physiological signal and/or the terminal device 110.

The monitoring object 140 refers to an object monitored by the device 150 for monitoring the physiological signal. For example, the monitoring object 140 may be a user of the device 150 for monitoring the physiological signal monitoring or a patient, an experimenter, etc., whose physiological signal needs to be collected.

The device 150 for monitoring the physiological signal refers to a device for collecting or monitoring the physiological signal of the monitoring object 140. The device 150 for monitoring the physiological signal may be worn on at least one body part (e.g., the chest, the back, the waist) of the monitoring object 140 to collect the physiological signal of the monitoring object 140. In some embodiments, the device 150 for monitoring the physiological signal may be a strap structure (e.g., a heart rate strap), including a strap and a plurality of electrodes. The strap may be configured to wear the device 150 for monitoring the physiological signal on at least one body part of the monitoring object. The plurality of electrodes may be arranged on an inner surface of the strap for attaching to the at least one body part of the monitoring object 140 to collect electrical signals (e.g., electric potentials at electrode positions) of the monitoring object 140.

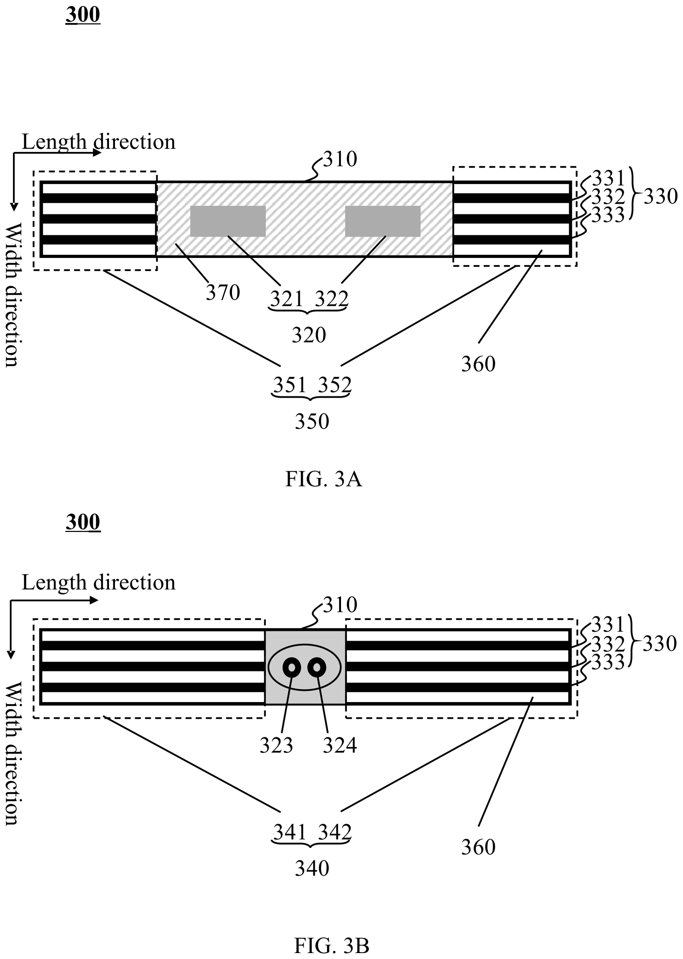

In some embodiments, the device 150 for monitoring the physiological signal may possess an independent power supply. The device 150 for monitoring the physiological signal may send collected data (e.g., the physiological signal) to other components (e.g., the storage device 130, or the terminal device 110) via a wired or wireless (e.g., Bluetooth, Wi-Fi, etc.) manner. In some embodiments, the one or more components on the application scenario 100 may be part of the device 150 for monitoring the physiological signal. For example, the device 150 for monitoring the physiological signal may include the storage device 130, etc. More descriptions regarding the device 150 for monitoring the physiological signal may be found in FIG. 3A to FIG. 3J and the related descriptions.

In some embodiments, the application scenario 100 may further include a processing device (not shown in FIG. 1, the processing device may be configured in the device 150 for monitoring the physiological signal or the terminal device 110). The processing device may obtain physiological data based on electrical signals collected by the plurality of electrodes. The physiological data refers to data that is determined based on the physiological signal and reflects a bioelectrical feature of the monitoring object (e.g., the monitoring object 140). For example, when the physiological signal is the electrocardiographic signal, the physiological data may be electrocardiogram data.

It should be noted that the above description regarding the application scenario 100 is merely for example and illustration, and does not limit the applicable scope of the present disclosure. For those skilled in the art, various modifications and changes to the application scenario 100 may be made under the guidance of the present disclosure. However, these modifications and changes are still within the scope of the present disclosure.

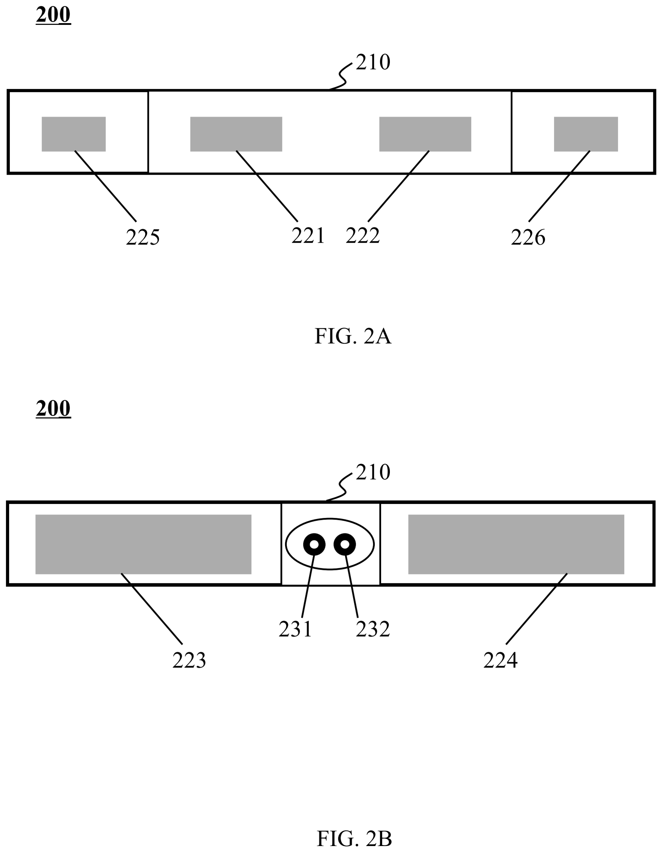

FIG. 2A is a diagram illustrating a structure of an inner surface of a traditional device for monitoring a physiological signal according to some embodiments of the present disclosure; FIG. 2B is a diagram illustrating a structure of an outer surface of a traditional device for monitoring a physiological signal according to some embodiments of the present disclosure. In some embodiments, as shown in FIG. 2A and FIG. 2B, a traditional device 200 for monitoring a physiological signal may include a strap 210 and a plurality of electrodes. The plurality of electrodes includes an electrode 221, an electrode 222, an electrode 223, an electrode 224, an electrode 225, and an electrode 226.

The strap 210 is a base structure of the traditional device 200 for monitoring the physiological signal. The strap 210 is configured to be worn on a body of a user (e.g., the monitoring object 140), so that the plurality of electrodes may attached to the body of the user. The strap 210 includes an inner surface (a surface contacting with the body of the user) and an outer surface (another surface opposite to the inner surface, which faces away from human skin). Connecting members (e.g., connection buckles, not shown in the figure) may be configured at two ends of the strap 210. When the connecting members at the two ends are connected (e.g., buckled), the strap 210 may surround and attach to a wearing part (e.g., the chest or the waist) of the user (e.g., the monitoring object 140).

A material of the strap 210 may include plant fibers (e.g., cotton fibers, hemp fibers, etc.), animal fibers (e.g., wool, etc.), and synthetic fibers (e.g., acrylic, polyester, etc.). For example, the strap 210 may be woven from the plant fibers.

The electrode 221 and the electrode 222 are electrical signal collection electrodes. The electrode 221 and the electrode 222 are not conductive to each other. As shown in FIG. 2A, the electrode 221 and the electrode 222 may be configured on the inner surface of the strap 210 and attach to the body of the monitoring object (e.g., the monitoring object 140). The electrode 221 and the electrode 222 are configured to collect electrical signals (e.g., potentials) at corresponding positions.

The electrode 221 and the electrode 222 may be conductive to a connection port 231 and a connection port 232, respectively. The connection port 231 and the connection port 232 may be connected to a transmitter (not shown in the figure). For example, the connection port 231 and the connection port 232 may be fastened and connected to the transmitter by a snap fastener. The transmitter may send the electrical signals (e.g., electrocardiogram potentials) collected by the electrode 221 and the electrode 222 to the processing device to determine the physiological signal (e.g., the electrocardiographic signal) and/or the physiological data (e.g., an electrocardiogram) of the monitoring object (e.g., the monitoring object 140) based on the electrical signals.

The electrode 223 and the electrode 224 are electrostatic reception electrodes. The electrode 223 and the electrode 224 are not conductive to each other. As shown in FIG. 2B, the electrode 223 and the electrode 224 may be configured on the outer surface of the strap 210. The electrode 223 and the electrode 224 are configured to receive an electrostatic charge from the exterior of the human body (e.g., the electrostatic charge generated by friction between the human body and clothing worn by the human body). In some embodiments, the electrode 223 and the electrode 224 may cover the electrode 221 and the electrode 222, respectively, to receive external electrostatic charges of the electrode 221 and the electrode 222 as much as possible.

The electrode 225 and the electrode 226 are electrostatic discharge electrodes. The electrode 225 and the electrode 226 are not conductive to each other. The electrode 225 is conductive to the electrode 223. The electrode 226 is conductive to the electrode 224. As shown in FIG. 2A, the electrode 225 and the electrode 226 may be configured on the inner surface of the strap 210 and attach to the body of the monitoring object (e.g., the monitoring object 140). The electrode 225 and the electrode 226 are configured to guide the electrostatic charges received by the electrode 223 and the electrode 224 into the body of the monitoring object (e.g., the monitoring object 140), respectively, to reduce an impact of the electrostatic charges on the electrical signals collected by the electrode 221 and the electrode 222, thereby achieving electrostatic shielding and improving signal quality of a finally obtained physiological signal.

A material of the electrode 221, the electrode 222, the electrode 223, the electrode 224, the electrode 225, and the electrode 226 may be non-elastic conductive rubber, etc.

However, the traditional device 200 for monitoring the physiological signal with the above structure has at least the following problems.

To enable the electrode 223 and the electrode 224 to cover the electrode 221 and the electrode 222, respectively, and due to the material of the electrode 223 and the electrode 224 being non-elastic rubber, an area of a non-elastic region on the traditional device 200 for monitoring the physiological signal is relatively large. This results in impairing overall elasticity, adjustability, and breathability of the traditional device 200 for monitoring the physiological signal, which may cause discomfort or even skin damage to the user (e.g., the monitoring object 140) during wearing, thereby affecting a wearing experience of the user. A reduced elasticity of the traditional device 200 for monitoring the physiological signal also leads to a poor fit between the traditional device 200 for monitoring the physiological signal and the human body, resulting in a low signal-to-noise ratio of the collected physiological signal.

The electrode 223 and the electrode 224 are located on the outer surface of the strap 210, which makes a manufacturing process of the traditional device 200 for monitoring the physiological signal more complex compared to a device for monitoring the physiological signal without an anti-static structure, thereby increasing a defect rate during production of the traditional device 200 for monitoring the physiological signal.

The presence of the electrode 225 and the electrode 226 increases an overall length of the traditional device 200 for monitoring the physiological signal, which causes the connecting members at the two sides of the strap 210 to move backward during wearing by the user. For example, a connecting member originally located at a side waist moves to a rear side of the waist during the wearing. The difficulty for the user to connect the connecting members is thereby increased, adversely affecting the user experience.

Based on this, embodiments of the present disclosure provide a device for monitoring the physiological signal (e.g., a device 300 for monitoring the physiological signal or a device 400 for monitoring the physiological signal). The device for monitoring the physiological signal achieves an anti-static effect while ensuring the user experience, and improves a signal-to-noise ratio of the electrocardiographic signal collected in an electrostatic scenario.

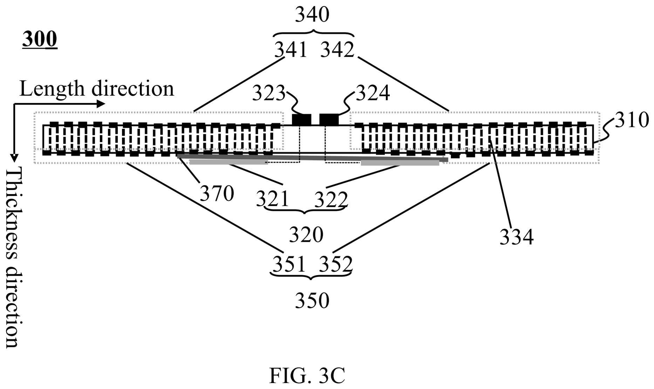

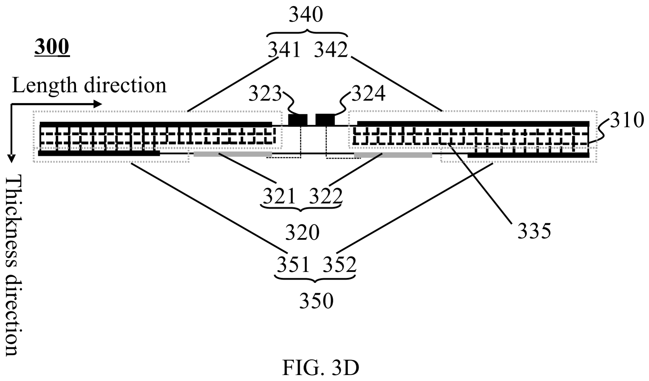

FIG. 3A is a diagram illustrating a structure of an inner surface of a device for monitoring a physiological signal according to some embodiments of the present disclosure; FIG. 3B is a diagram illustrating a structure of an outer surface of a device for monitoring a physiological signal according to some embodiments of the present disclosure; FIG. 3C is a diagram illustrating a front view of a device for monitoring a physiological signal according to some embodiments of the present disclosure; FIG. 3D is a diagram illustrating another front view of a device for monitoring a physiological signal according to some embodiments of the present disclosure. FIG. 3C and FIG. 3D are front views of the device for monitoring the physiological signal when placed flat on a table or ground (e.g., when the outer surface or the inner surface is placed parallel to the table/ground).

In some embodiments, as shown in FIG. 3A to FIG. 3D, the device 300 for monitoring the physiological signal may include a strap 310 and a plurality of electrodes 320. One or more conductive threads 330 (e.g., a first conductive thread 331, a second conductive thread 332, a third conductive thread 333, etc.) are arranged on the strap 310. A first portion 340 of the one or more conductive threads 330 is located on an outer surface of the strap 310 or between an inner surface and the outer surface of the strap 310. A second portion 350 (including a left second portion 351 and a right second portion 352) of the one or more conductive threads 330 extends to the inner surface of the strap 310 and electrically connects the first portion 340 of the one or more conductive threads 330 to a body of the user to provide electrostatic shielding for the plurality of electrodes 320.

The inner surface of the strap 310 refers to a surface that contacts the body of the user (e.g., a surface that contacts the skin of the user). The outer surface of the strap 310 refers to another surface opposite to the inner surface (i.e., a surface facing away from the human skin).

The strap 310 refers to a base structure of the device for monitoring the physiological signal. The strap 310 is configured to be worn on the body of the user (e.g., the monitoring object 140), so that the plurality of electrodes may attach to the body of the user. For example, connection buckles may be configured at two ends of the strap 310. When the connection buckles at the two ends are buckled, the strap 310 may surround and attach to a wearing part of the monitoring object 140 (e.g., surround and attach to the chest or waist of the user).

A material of the strap 310 may include plant fibers (e.g., cotton fibers, hemp fibers, etc.), animal fibers (e.g., wool, etc.), synthetic fibers (e.g., acrylic, polyester, etc.), etc. For example, the strap 310 may be woven from the cotton fibers.

In some embodiments, the one or more conductive threads may include metal fibers, carbon fibers, compound fibers, or polymer fibers.

In some embodiments, the strap 310 is at least formed by weaving elastic threads (e.g., cotton threads), and the one or more conductive threads 330 (e.g., silver fiber threads) are interwoven through the inner surface and the outer surface of the strap 310. As shown in FIG. 3A and FIG. 3B, the strap 310 may include an elastic portion 360 (the white parts in the figure) composed of the elastic threads and a plurality of conductive threads 330 (the black parts in the figure, including the first conductive thread 331, the second conductive thread 332, and the third conductive thread 333).

In some embodiments, as shown in FIG. 3C, the plurality of conductive threads included in the one or more conductive threads 330 (e.g., the first conductive thread 331, the second conductive thread 332, and the third conductive thread 333) may be interwoven through the inner surface and the outer surface of the strap 310, respectively, to form the first portion 340 and the second portion 350. The first portion 340 includes a portion of the one or more conductive threads 330 located on the outer surface of the strap 310 and a portion located between the outer surface and the inner surface of the strap 310. For example, the first portion 340 includes portions of the first conductive thread 331, the second conductive thread 332, and the third conductive thread 333 that are located on the outer surface of the strap 310 and portions located between the outer surface and the inner surface of the strap 310. The second portion 350 includes a portion located on the inner surface of the strap 310. For example, the second portion 350 includes portions of the first conductive thread 331, the second conductive thread 332, and the third conductive thread 333 that are located on the inner surface of the strap 310. A portion of the one or more conductive threads 330 located between the inner surface and the outer surface of the strap 310 is referred to as a first internal conductive thread, e.g., a first internal conductive thread 334 in FIG. 3C. An internal conductive thread (e.g., the first internal conductive thread 334) of the one or more conductive threads 330 may electrically connect a portion of the one or more conductive threads 330 located on the inner surface of the strap 310 with a portion of the one or more conductive threads 330 located on the outer surface of the strap 310 to achieve the electrostatic shielding.

To achieve the structure of the device 300 for monitoring the physiological signal described in the above embodiments, a weaving process of the device 300 for monitoring the physiological signal may be as follows. First, the strap 310 is formed by weaving based on the elastic threads, then, each of the plurality of conductive threads included in the one or more conductive threads 330 (e.g., the first conductive thread 331, the second conductive thread 332, and the third conductive thread 333) is interwoven through the inner surface and the outer surface of the strap.

A specific process of interwoven-weaving may include weaving the plurality of conductive threads included in the one or more conductive threads 330 in parallel. For example, the first conductive thread 331, the second conductive thread 332, and the third conductive thread 333 are interwoven along a length direction of the strap 310 with a certain spacing (an equal spacing or an unequal spacing) in a width direction of the strap 310. The length of each conductive thread used is sufficient for weaving from the head to the tail along the length direction of the strap 310. Thus, the one or more conductive threads 330 are obtained. The one or more conductive threads 330 include three conductive threads spaced apart side by side along the width direction of the strap 310, i.e., the first conductive thread 331, the second conductive thread 332, and the third conductive thread 333 spaced apart side by side in the width direction of the strap 310.

The electrostatic shielding refers to a state where the electrode of the device for monitoring the physiological signal is not affected by an external charge or an electric field (e.g., an electrostatic field). For example, according to a structure of the device 300 for monitoring the physiological signal as shown in FIG. 3C, when static electricity exists on the outer surface of the strap 310, electrostatic charges may sequentially enter the human body via the first portion 340 and the second portion 350 of the conductive thread 330, preventing the electrostatic charges from affecting the plurality of electrodes 320. The first portion 340 is a portion located on the outer surface of the strap 310, e.g., the portion of the first conductive thread 331 located on the outer surface of the strap 310, the first internal conductive thread 334. The second portion 350 is a portion of the one or more conductive threads 330 located on the inner surface of the strap 310, e.g., the portion of the first conductive thread 331 located on the inner surface of the strap 310.

In some embodiments of the present disclosure, using the elastic threads to weave the strap can ensure that the strap possess good elasticity and softness, thereby improving user wearing comfort. The one or more conductive threads are interwoven through the inner surface and the outer surface of the strap, which allows the one or more conductive threads to form a conductive region on the outer surface to disperse the static electricity and introduce the static electricity into the human body, thereby achieving the electrostatic shielding for the electrodes.

In some embodiments, the strap 310 may be formed at least by mixed weaving of the elastic threads and the one or more conductive threads. The mixed weaving refers to a process where the one or more conductive threads are mixed into the elastic threads at a certain ratio (e.g., 1:5, 2:3, etc.) to form an integral unit, thereby weaving the strap.

As shown in FIG. 3D, according to the mixed weaving of the strap 310 described above, the plurality of conductive threads 330 (e.g., the first conductive thread 331, the second conductive thread 332, and the third conductive thread 333) may include the first portion 340 and the second portion 350. The first portion 340 includes a portion of the one or more conductive threads 330 located on the outer surface of the strap 310 and a portion of the one or more conductive threads 330 located between the outer surface and the inner surface of the strap 310 (e.g., the portions of the first conductive thread 331, the second conductive thread 332, and the third conductive thread 333 located on the outer surface of the strap 310 and portions located between the outer surface and the inner surface of the strap 310). The second portion 350 includes a portion of the one or more conductive threads 330located on the inner surface of the strap 310 (e.g., the portions of the first conductive thread 331, the second conductive thread 332, and the third conductive thread 333 located on the inner surface of the strap 310). The portion of the one or more conductive threads 330 located between the inner surface and the outer surface of the strap 310 is referred to as a second internal conductive thread, e.g., a second internal conductive thread 335 in FIG. 3D. The internal conductive thread (e.g., the second internal conductive thread 335) of the one or more conductive threads 330 may electrically connect the portion of the one or more conductive threads 330 located on the inner surface of the strap 310 with the portion of the one or more conductive threads 330 located on the outer surface of the strap 310 to achieve the electrostatic shielding. Each internal conductive thread corresponds to one conductive channel, and each conductive channel may independently and electrically connect the portion of the corresponding conductive thread 330 located on the inner surface of the strap 310 with the portion of the corresponding conductive thread 330 located on the outer surface of the strap 310.

In some embodiments of the present disclosure, when a count of the conductive channels is small, the breakage of the conductive thread at the conductive channel may affect the electrostatic shielding effect. Under the mixed weaving of the elastic threads and the conductive threads, since the conductive threads are distributed in various layers of the strap (the weaving process causes the strap to form a plurality of layered structures during the weaving), the count of conductive channels between the inner surface and the outer surface is effectively increased, thereby better guiding the static electricity from the outer surface into the human body, and improving the stability of the electrostatic shielding.

In some embodiments, at least a portion of the strap 310 may be formed by weaving the one or more conductive threads. The one or more conductive threads penetrate through the inner surface and the outer surface of the strap 310 in a thickness direction of the strap 310. In some embodiments, a certain partial region of the strap 310 (e.g., a region other than a position on the strap 310 where the electrode 320 is located) may be formed by weaving the one or more conductive threads. In this way, the one or more conductive threads may electrically connect the inner surface and the outer surface of the strap 310 at any position in the partial region, which allows the partial region formed by weaving the one or more conductive threads to function as a conductive region as a whole, thereby better guiding the static electricity of the outer surface into the human body and improving the stability of the electrostatic shielding. In some embodiments, the partial region formed by weaving the one or more conductive threads may also be elastic. Merely by way of example, an elastic thread with good elasticity may be prepared, the one or more conductive threads are wound around a peripheral side of the elastic thread (similar to a spring winding manner) to obtain an elastic conductive thread. Subsequently, the elastic conductive thread is woven to form at least a partial region of the strap 310. In some embodiments, the strap 310 may also be woven from the elastic conductive thread obtained by the above manner.

In some embodiments, the one or more conductive threads may not electrically connect the inner surface and the outer surface of the strap. For example, the one or more conductive threads are only interwoven on the outer surface of the strap and do not extend to the inner surface. In this case, the one or more conductive threads only form the conductive region on the outer surface to disperse the static electricity.

In some embodiments, the plurality of electrodes 320 included in the device 300 for monitoring the physiological signal are arranged on the inner surface of the strap 310 and are configured to receive electrical signals from the body of the user.

Exemplarily, as shown in FIG. 3A and FIG. 3B, the plurality of electrodes 320 are arranged on the inner surface of the strap 310 and are configured to receive the electrical signals (e.g., electrocardiographic signals) from the body of the user. As shown in FIG. 3C or FIG. 3D, the first portion 340 of the one or more conductive threads 330 arranged on the strap 310 is located on the outer surface or between the inner surface and the outer surface, such that each electrode of the plurality of electrodes (e.g., a first electrode 321 or a second electrode 322) is located at least partially between the first portion 340 of the one or more conductive threads 330 and the body of the user. As shown in FIG. 3A, the second portion 350 of the one or more conductive threads 330 extends to the inner surface and electrically connects the first portion 340 of the one or more conductive threads 330 to the body of the user to achieve the electrostatic shielding for the plurality of electrodes 320 (e.g., the first electrode 321 and the second electrode 322).

The plurality of electrodes refers to electrodes for collecting the physiological signal (e.g., the electrocardiographic signal). A material of the plurality of electrodes may be a conductive material with low hardness. For example, the material of the plurality of electrodes may be conductive rubber, conductive silicone, etc.

In some embodiments, the plurality of electrodes 320 are attached to the strap 310 via one or more insulating layers, and the one or more insulating layers provide insulation between the plurality of electrodes 320 and the one or more conductive threads 330. For example, as shown in FIG. 3A, insulating layers 370 may be configured between the plurality of electrodes 320 and the strap 310, so as to insulate the plurality of electrodes 320 from the strap 310 (including the one or more conductive threads 330 in the strap 310). A material of the one or more insulating layers is any reasonable insulating material. For example, the material of the one or more insulating layers may be insulating rubber, etc.

In some embodiments, a plurality of holes may be provided in the strap 310, and each electrode of the plurality of electrodes 320 may be configured in one of the plurality of holes. An insulating material may be filled between the each electrode of the plurality of electrodes 320 and an inner wall of each of the plurality of holes, so as to insulate the plurality of electrodes from the strap (including the one or more conductive threads in the strap). In some embodiments, the one or more conductive threads may not be woven on the electrodes 320 and a certain region nearby (i.e., the one or more conductive threads are interwoven in regions other than the electrodes 320), thereby achieving insulation between the electrodes 320 and the strap 310. In this case, the electrodes 320 may be directly attached to the strap 310. In some embodiments, insulating substances may cover the one or more conductive threads at locations on the strap 310 where the electrodes 320 are located. The insulating substances provide the insulation between the electrodes 320 and the strap 310. For example, the strap may first be woven with bare conductive threads, and then post-processing (an insulation treatment) is performed on the bare conductive threads in a strap region where the electrodes 320 are located to achieve the insulation. As another example, conductive threads whose surfaces are covered with the insulating substances may be used to weave the strap, and then post-processing (a de-insulation treatment) is performed on the conductive threads in regions of the strap other than where the electrodes 320 are located to remove the insulating substances.

In some embodiments of the present disclosure, by insulating the electrodes from the one or more conductive threads, it can be ensured that the one or more conductive threads do not affect the physiological signal obtained by the electrodes from the body of the user.

In some embodiments, the device 300 for monitoring the physiological signal may further be configured with a plurality of connection ports on the outer surface of the strap. Each of the plurality of connection ports may be respectively electrically connected with each electrode of the plurality of electrodes. The plurality of connection ports may be connected to the transmitter (e.g., connected by a snap fastener). The transmitter may send the electrical signals (e.g., the electrocardiographic potentials) collected by the plurality of electrodes to the processing device. The processing device may determine the physiological signal (e.g., the electrocardiographic signal) and/or the physiological data (e.g., the electrocardiogram) of the monitoring object (e.g., the monitoring object 140) based on the electrical signals.

Exemplarily, as shown in FIG. 3B, the device 300 for monitoring the physiological signal may further be configured with a connection port 323 and a connection port 324 on the outer surface of the strap 310. The first electrode 321 and the second electrode 322 may be electrically connected to the connection port 323 and the connection port 324, respectively. The connection port 323 and the connection port 324 may be connected to the transmitter (not shown in the figure), e.g., connected by the snap fastener. The transmitter may send the electrical signals (e.g., the electrocardiographic potentials) obtained by the first electrode 321 and the second electrode 322 to the processing device, and the processing device may determine the physiological signal (e.g., the electrocardiographic signal) and/or the physiological data (e.g., the electrocardiogram) of the monitoring object (e.g., the monitoring object 140) based on the electrical signals.

In some embodiments, both the first portion and the second portion of the one or more conductive threads include a plurality of conductive channels arranged side by side in the width direction of the strap, respectively.

The conductive channels refer to channels through which electric charges (e.g., the electrostatic charges) may flow. For example, each conductive thread in the strap may correspondingly form a conductive channel. The count of the conductive channels may be preset by a designer or a producer of the device for monitoring the physiological signal. When the device for monitoring the physiological signal is producing, conductive threads with the above count are woven into the strap to obtain the conductive channels with the above count. Exemplarily, the count of the conductive channels may be 3, 4, 5, etc.

For example, as shown in FIG. 3A and FIG. 3B, both the first portion 340 and the second portion 350 of the one or more conductive threads 330 include 3 conductive threads arranged side by side in the width direction of the strap 310: the first conductive thread 331, the second conductive thread 332, and the third conductive thread 333. The first conductive thread 331, the second conductive thread 332, and the third conductive thread 333 respectively correspond to a conductive channel. Based on this, both the first portion 340 and the second portion 350 of the one or more conductive threads 330 include 3 conductive channels arranged side by side in the width direction of the strap 310.

In some embodiments of the present disclosure, by forming the plurality of conductive channels arranged side by side in the width direction of the strap, each conductive channel of the plurality of conductive channels can guide static electricity from the outer surface into the human body, thereby achieving the electrostatic shielding for the electrodes.

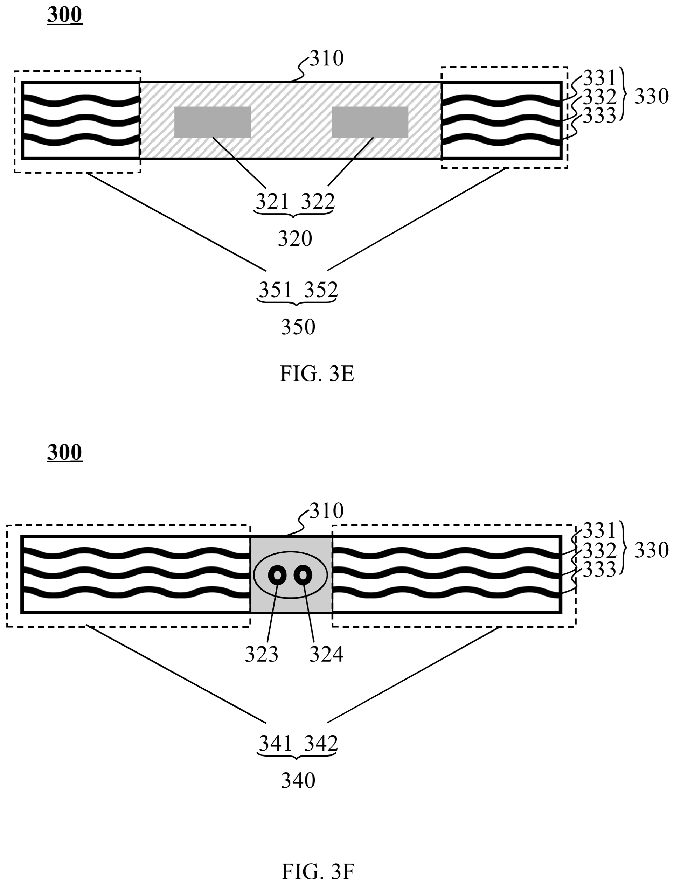

FIG. 3E is a diagram illustrating another structure of an inner surface of a device for monitoring a physiological signal according to some embodiments of the present disclosure; and FIG. 3F is a diagram illustrating another structure of an outer surface of a device for monitoring a physiological signal according to some embodiments of the present disclosure.

In some embodiments, each of a plurality of conductive channels is formed by weaving one or more conductive threads in a wave pattern along a length direction of a strap.

As shown in FIG. 3E and FIG. 3F, each of the one or more conductive threads 330 of the strap 310 (e.g., the first conductive thread 331, the second conductive thread 332, and the third conductive thread 333) is respectively formed by weaving in the wave pattern along the length direction of the strap 310. Based on this, the conductive channel corresponding to the each conductive thread 330 is bent in the wave pattern along the length direction of the strap 310.

In some embodiments, each of the plurality of conductive channels in the length direction of the strap may also be a Z-shaped line, a right-angle wave line, etc., which is not limited herein.

In some embodiments of the present disclosure, weaving the conductive threads (or the conductive channels) in the wave pattern can reserve a stretching displacement for the conductive threads, which ensures that the conductive channels with the wave pattern formed by the conductive threads are stretchable in the length direction, so as to cause the conductive threads less prone to breakage when the strap is stretched. Thus, the overall strap is ensured to have good elasticity and extensibility.

In some embodiments, as shown in FIG. 3A, the plurality of electrodes 320 include the first electrode 321 and the second electrode 322 spaced apart along the length direction of the strap 310. In some embodiments, as shown in FIG. 3B, the first portion 340 of the one or more conductive thread 330 forms a first conductive region 341 and a second conductive region 342 insulated from each other, the first conductive region 341 covers an outer side of the first electrode 321 and is insulated from the first electrode 321, and the second conductive region 342 covers an outer side of the second electrode 322 and is insulated from the second electrode 322.

Covering refers to that a projection region of an electrode is contained within a projection region of a corresponding conductive region. The projection region refers to a region corresponding to a projection of the conductive region or the electrode in the thickness direction of the strap.

In some embodiments, the first conductive region covers the first electrode in the length direction of the strap, and the second conductive region covers the second electrode in the length direction of the strap.

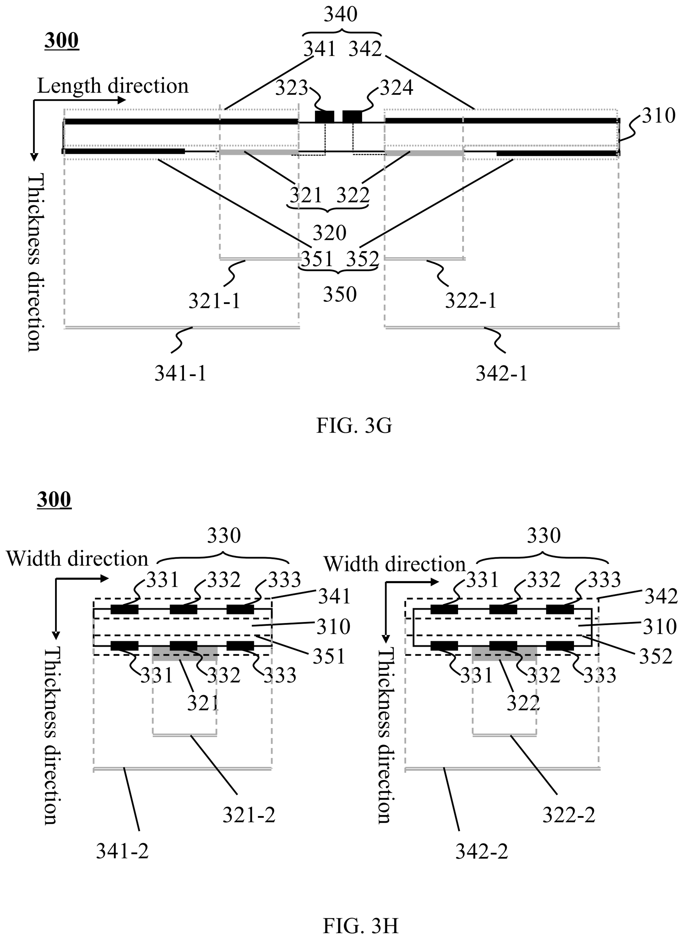

FIG. 3G is a schematic diagram illustrating a front projection view of a device for monitoring a physiological signal according to some embodiments of the present disclosure.

Exemplarily, as shown in FIG. 3G, a projection long side 321-1 of the first electrode 321 (a side of a projection region of the first electrode 321 that is parallel to the length direction of the strap 310) is contained within a projection long side 341-1 of the first conductive region 341 (a side of a projection region of the first conductive region 341 that is parallel to the length direction of the strap 310). A projection long side 322-1 of the second electrode 322 (a side of a projection region of the second electrode 322 that is parallel to the length direction of the strap 310) is contained within a projection long side 342-1 of the second conductive region 342 (a side of a projection region of the second conductive region 342 that is parallel to the length direction of the strap 310). This arrangement causes the first conductive region 341 to cover the first electrode 321 in the length direction of the strap 310 and the second conductive region 342 to cover the second electrode 322 in the length direction of the strap 310. Taking the projection long side 321-1 of the first electrode 321 being contained within the projection long side 341-1 of the first conductive region 341 as an example, containing refers to that a length of the projection long side 321-1 of the first electrode 321 is not greater than a length of the projection long side 341-1 of the first conductive region 341, and the projection long side 321-1 of the first electrode 321 is spatially located within the projection long side 341-1 of the first conductive region 341. The containing relationship described later is the same.

In some embodiments of the present disclosure, by arranging the conductive region to cover the corresponding electrode in the length direction, the conductive region can disperse the static electricity near the outer surface directly opposite the electrode, which can weaken or eliminate an effect of the static electricity on the electrode to a maximum extent.

In some embodiments, the first conductive region covers the first electrode in the width direction of the strap, and the second conductive region covers the second electrode in the width direction of the strap 310.

FIG. 3H is a schematic diagram illustrating a side projection view of a device for monitoring a physiological signal according to some embodiments of the present disclosure. A left diagram and a right diagram in FIG. 3H are schematic diagrams illustrating side projection views observed from two opposite sides of the device 300 for monitoring the physiological signal, respectively.

Exemplarily, as shown in FIG. 3H, a projection wide side 321-2 of the first electrode 321 (a side of the projection region of the first electrode 321 that is parallel to the width direction of the strap 310) is contained within a projection wide side 341-2 of the first conductive region 341 (a side of the projection region of the first conductive region 341 that is parallel to the width direction of the strap 310). A projection wide side 322-2 of the second electrode 322 (a side of the projection region of the second electrode 322 that is parallel to the width direction of the strap 310) is contained within a projection wide side 342-2 of the second conductive region 342 (a side of the projection region of the second conductive region 342 that is parallel to the width direction of the strap 310). This arrangement causes the first conductive region 341 to cover the first electrode 321 in the width direction of the strap 310 and the second conductive region 342 to cover the second electrode 322 in the width direction of the strap 310.

In some embodiments of the present disclosure, by arranging the conductive region to cover the corresponding electrode in the width direction, the conductive region can disperse the static electricity near the outer surface directly opposite the electrode, which can weaken or eliminate an effect of the static electricity on the electrode to the maximum extent.

In some embodiments of the present disclosure, static electricity that has a greatest effect on the electrode is the static electricity on the outer surface directly opposite the electrode and the static electricity near the outer surface. By arranging the conductive region to cover the corresponding electrode, the conductive region can disperse the static electricity near the outer surface directly opposite the electrode, which can weaken or eliminate the effect of the static electricity on the electrode to the maximum extent.

In some embodiments, the first portion 340 of the one or more conductive threads 330 may form a conductive region, and the conductive region covers the first electrode 321 and the second electrode 322 in the length direction of the strap. With reference to FIG. 3B, the first conductive region 341 and the second conductive region 342 corresponding to the first portion 340 of the one or more conductive threads 330 may be electrically connected to form a conductive region corresponding to the first portion 340. The conductive region corresponding to the first portion 340 covers the first electrode 321 and the second electrode 322 in the length direction of the strap 310.

In some embodiments, the conductive region corresponding to the first portion of the one or more conductive threads covers the first electrode and the second electrode in the length direction.

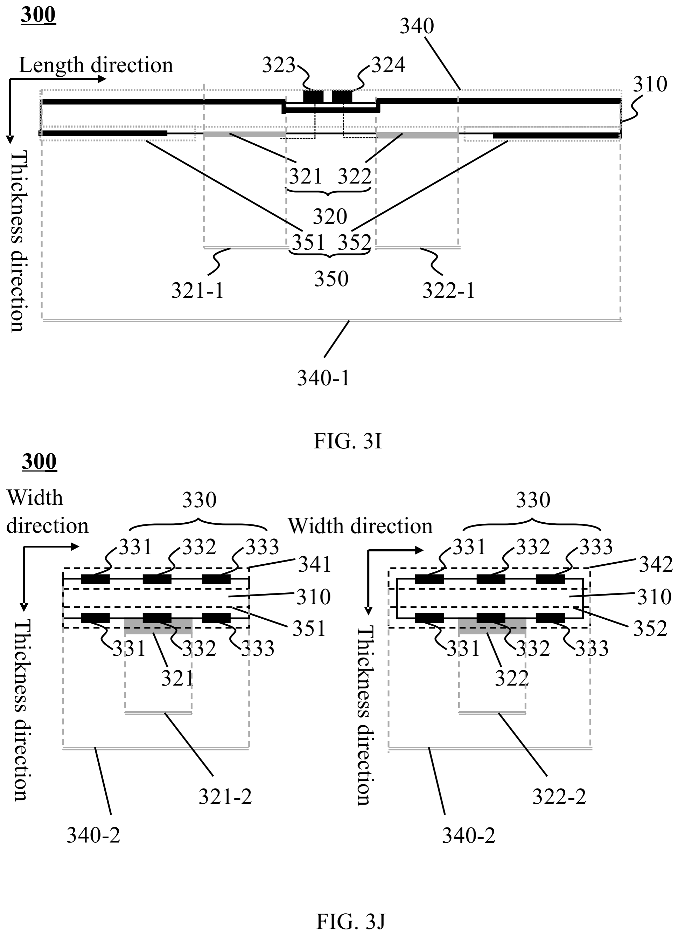

FIG. 3I is a schematic diagram illustrating another front projection view of a device for monitoring a physiological signal according to some embodiments of the present disclosure.

Exemplarily, as shown in FIG. 3I, the projection long side 321-1 of the first electrode 321 and the projection long side 322-1 of the second electrode 322 are both contained within a projection long side 340-1 of the first portion 340 of the one or more conductive threads 330 (a side of the projection region of the first portion 340 that is parallel to the length direction of the strap 310). This arrangement causes the conductive region corresponding to the first portion 340 of the one or more conductive threads 330 to cover the first electrode 321 and the second electrode 322 in the length direction of the strap 310.

In some embodiments, the conductive region formed by the first portion 340 of the one or more conductive threads 330 covers the first electrode and the second electrode in the width direction of the strap.

FIG. 3J is a schematic diagram illustrating another side projection view of a device for monitoring a physiological signal according to some embodiments of the present disclosure. A left diagram and a right diagram in FIG. 3J are schematic diagrams illustrating side projection views observed from two opposite sides of the device 300 for monitoring the physiological signal, respectively.

Exemplarily, as shown in FIG. 3J, the projection wide side 321-2 of the first electrode 321 and the projection wide side 322-2 of the second electrode 322 are both contained within a projection wide side 340-2 of the first portion 340 of the one or more conductive threads 330 (a side of the projection region of the first portion 340 that is parallel to the width direction of the strap 310). This arrangement causes the conductive region to cover the first electrode 321 and the second electrode 322 in the width direction of the strap 310.

In some embodiments of the present disclosure, by arranging the conductive region to cover the two electrodes in the width direction, the conductive region can disperse static electricity near the outer surfaces directly opposite the two electrodes, which can weaken or eliminate an effect of the static electricity on the electrodes to a maximum extent.

In some embodiments of the present disclosure, by electrically connecting the two conductive regions to form a single conductive region, an ability of the conductive region to disperse static electricity can be further enhanced. By arranging the conductive region to cover the two electrodes in the length direction, an effect of the static electricity on the electrodes can be weakened or eliminated to the maximum extent.

It should be noted that, since the second portion of the one or more conductive threads electrically connects the first portion of the one or more conductive threads to the body of the user, when the two conductive regions are electrically connected to form a single conductive region, a circuit may be formed between the one or more conductive threads and the body of the user. This arrangement may affect a potential difference between two sides of the body, thereby affecting the physiological signal collected by the first electrode and/or the second electrode from the body of the user.

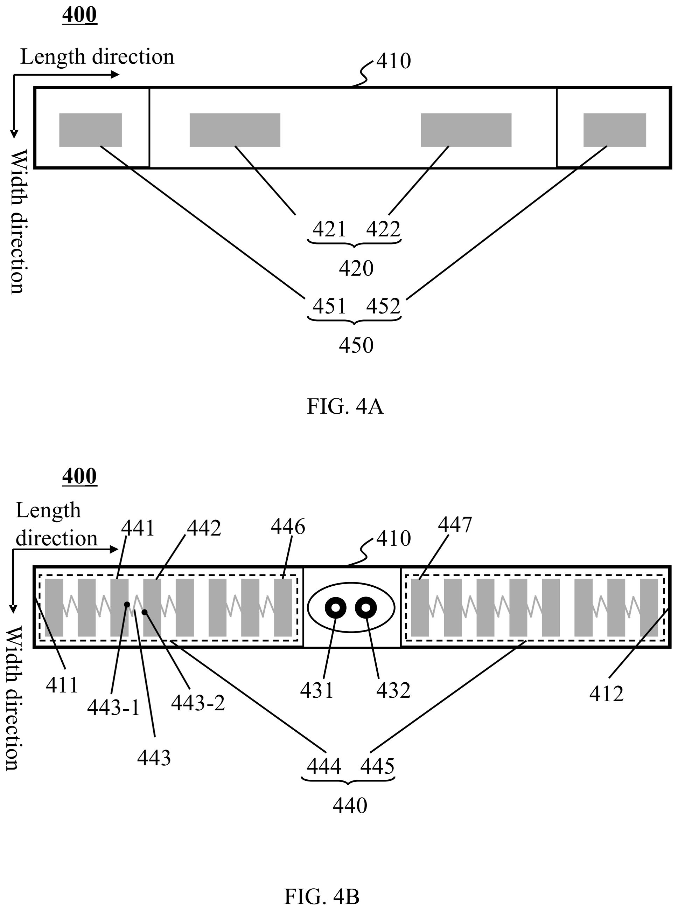

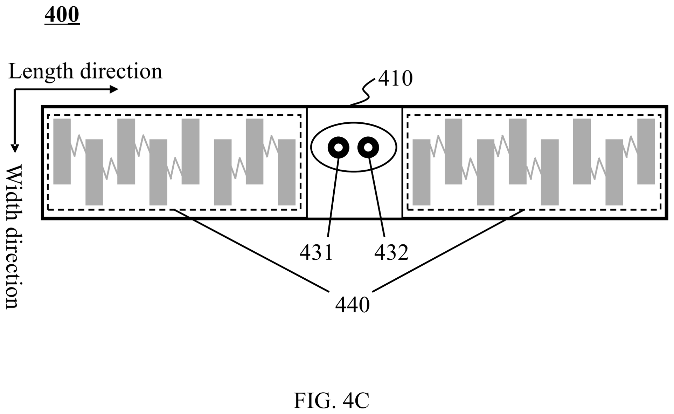

FIG. 4A is a diagram illustrating a structure of an inner surface of another device for monitoring a physiological signal according to some embodiments of the present disclosure; FIG. 4B is a diagram illustrating a structure of an outer surface of another device for monitoring a physiological signal according to some embodiments of the present disclosure; and FIG. 4C is a diagram illustrating another structure of an outer surface of another device for monitoring a physiological signal according to some embodiments of the present disclosure.

In some embodiments, as shown in FIG. 4A and FIG. 4B, a device 400 for monitoring the physiological signal may include a strap 410, a plurality of signal electrodes 420, two sets of electrostatic protection sheets 440, and a ground electrode 450.

Similar to the strap 210 or the strap 310, the strap 410 refers to a base structure of the device 400 for monitoring the physiological signal. The strap 410 is configured to be worn on a body of a user (e.g., the monitoring object 140), so that the plurality of electrodes may attach to the body of the user. For example, two ends of the strap 410 may be configured with connection buckles. When the connection buckles at the two ends are buckled, the strap 410 may surround and attach to a wearing part of the monitoring object 140 (e.g., surround and attach to the chest of the user).

Similar to the strap 310, a material of the strap 410 may include plant fibers (e.g., cotton fibers, hemp fibers, etc.), animal fibers (e.g., wool, etc.), synthetic fibers (e.g., acrylic, polyester, etc.), etc. For example, the strap may be woven from the cotton fibers.

The plurality of signal electrodes are arranged on an inner surface of the strap and are configured to receive electrical signals (e.g., electrocardiographic signals) from the body of the user. A material of the signal electrode may be a conductive material with low hardness, e.g., the conductive rubber, the conductive silicone, etc. The plurality of signal electrodes are not electrically connected to each other. Exemplarily, as shown in FIG. 4A, the plurality of signal electrodes 420 are arranged on the inner surface of the strap 410 and are configured to receive the electrical signals (e.g., the electrocardiographic signals) from the body of the user.

In some embodiments, the device for monitoring the physiological signal may also be configured with a plurality of connection ports on an outer surface of the strap, and each of the plurality of connection ports may be electrically connected to each of the plurality of signal electrodes of the plurality of signal electrodes, respectively. The plurality of connection ports may be connected to a transmitter (e.g., connected by a snap fastener). The transmitter may send the electrical signals (e.g., the electrocardiographic potentials) collected by the plurality of signal electrodes to a processing device. The processing device may determine the physiological signal (e.g., the electrocardiographic signal) and/or the physiological data (e.g., the electrocardiogram) of the monitoring object (e.g., the monitoring object 140) based on the electrical signals.

Exemplarily, as shown in FIG. 4B, the device 400 for monitoring the physiological signal may also be configured with a connection port 431 and a connection port 432 on an outer surface of the strap 410. A first signal electrode 421 and a second signal electrode 422 included in the plurality of signal electrodes 420 may be electrically connected to the connection port 431 and the connection port 432, respectively. The connection port 431 and the connection port 432 may be connected to the transmitter (not shown in the figure), e.g., connected by the snap fastener. The transmitter may send the electrical signals (e.g., the electrocardiographic potentials) collected by the first signal electrode 421 and the second signal electrode 422 to the processing device. The processing device may determine the physiological signal (e.g., the electrocardiographic signal) and/or the physiological data (e.g., the electrocardiogram) of the monitoring object (e.g., the monitoring object 140) based on the electrical signals.

The two sets of electrostatic protection sheets may be disposed on the outer surface of the strap 410 or be disposed between the inner surface and the outer surface of the strap 410, which cause the plurality of signal electrodes 420 to be located between the electrostatic protection sheets and the body of the user (e.g., the body of the monitoring object 140). In some embodiments, a first set of the two sets of electrostatic protection sheets may be disposed on a first region of the outer surface of the strap 410 or be disposed between the inner surface and the first region of the outer surface, and a second set of the two sets of electrostatic protection sheets may be disposed on a second region of the outer surface of the strap 410 or be disposed between the inner surface and the second region of the outer surface. The first region of the outer surface refers to a left region on the strap. For example, the first region may be a region between a first end 411 of the strap 410 and the connection port 431. The second region of the outer surface refers to a right region on the strap. For example, the second region may be a region between a second end 412 of the strap 410 and the connection port 432. The first set of electrostatic protection sheets includes a plurality of electrostatic protection sheets. The second set of electrostatic protection sheets includes a plurality of electrostatic protection sheets. Exemplarily, as shown in FIG. 4B, a first set of electrostatic protection sheets 440 of the device for monitoring the physiological signal is disposed on the first region of the outer surface of the strap 410. A second set of electrostatic protection sheets 440 is disposed on the second region of the outer surface of the strap 410. The plurality of electrostatic protection sheets 440 cover the plurality of signal electrodes 420 in a thickness direction of the strap 410. This arrangement causes the plurality of signal electrodes 420 (e.g., the first signal electrode 421 and the second signal electrode 422) to be located between the plurality of electrostatic protection sheets 440 and the body of the user.

The electrostatic protection sheets refer to conductive sheets used to receive electrostatic charges on an outer surface of the device for monitoring the physiological signal (e.g., electrostatic charges generated by friction between the human body and clothing worn by the human body). A material of the electrostatic protection sheets may be a conductive material with low hardness, e.g., the conductive rubber, the conductive silicone, etc.

In some embodiments, the plurality of electrostatic protection sheets are arranged side by side along a length direction of the strap.

In some embodiments, as shown in FIG. 4B, the plurality of electrostatic protection sheets 440 are arranged side by side along a length direction of the strap 410. The side-by-side arrangement refers that a reference point of each electrostatic protection sheet of the plurality of electrostatic protection sheets 440 is located on a same reference line. The reference point of the each electrostatic protection sheet may be preset (e.g., preset as a geometric center point of the each electrostatic protection sheet). The reference line refers to a straight line in the strap 410 that is parallel to the length direction of the strap 410 (e.g., a center line in the strap 410 that is parallel to the length direction of the strap 410). A shape of the each electrostatic protection sheet of the plurality of electrostatic protection sheets 440 may be pre-selected (e.g., as shown in FIG. 4B, the each electrostatic protection sheet is rectangular). A length of the each electrostatic protection sheet of the plurality of electrostatic protection sheets 440 in the length direction of the strap 410 may be the same or different. A length of the each electrostatic protection sheet of the plurality of electrostatic protection sheets 440 in a width direction of the strap 410 may also be the same or different.

In some embodiments, as shown in FIG. 4C, the plurality of electrostatic protection sheets 440 are staggered along the length direction of the strap 410. The staggered arrangement refers that reference points of at least a portion of the electrostatic protection sheets of the plurality of electrostatic protection sheets 440 are not located on the same reference line. In some embodiments, the plurality of electrostatic protection sheets 440 may not only be arranged side by side in the length direction, but may also be arranged side by side in the width direction, thereby forming a matrix distribution. In this configuration, a size of the each electrostatic protection sheet is reduced in the length direction and/or the width direction by increasing a quantity, thereby improving softness or elasticity of the strap.

In some embodiments, electrostatic protection sheets 440 located at different positions may have a same size or distribution density. In some embodiments, the electrostatic protection sheets 440 located at different positions may have different sizes or distribution densities. For example, a plurality of electrostatic protection sheets at the outer surface directly facing the electrode 320 may have a larger size or a denser distribution to improve an electrostatic shielding effect of the electrostatic protection sheets on the electrode. The electrostatic protection sheets at other regions of the outer surface may have a smaller size or a sparser distribution to ensure that the strap 410 (especially buckle regions at the two ends of the strap) has good elasticity.

In some embodiments, at least a portion of the electrostatic protection sheets of the plurality of electrostatic protection sheets are electrically connected to each other via the one or more conductive threads.

Exemplarily, as shown in FIG. 4B, an electrostatic protection sheet 441 and an electrostatic protection sheet 442 of the plurality of electrostatic protection sheets 440 are connected to each other via a conductive thread 443 to achieve a conduction between the electrostatic protection sheet 441 and the electrostatic protection sheet 442.

In some embodiments of the present disclosure, the at least a portion of the electrostatic protection sheets of the plurality of electrostatic protection sheets are electrically connected to each other via the one or more conductive threads, so that the plurality of electrostatic protection sheets can form a large conductive region, which facilitates dispersion of static electricity near the outer surface directly facing the signal electrode, thereby minimizing or eliminating an effect of the static electricity on the electrode.

In some embodiments, a distance between connection points on a conductive thread corresponding to two adjacent electrostatic protection sheets is less than a natural length of the conductive thread.

Exemplarily, as shown in FIG. 4B, a distance between connection points (a connection point 443-1 and a connection point 443-2) on the conductive thread 443 corresponding to adjacent electrostatic protection sheets 441 and 442 is less than a natural length of the conductive thread 443.

To achieve that the distance between the connection points of the two adjacent electrostatic protection sheets and the one of the one or more conductive threads connecting the two adjacent electrostatic protection sheets is less than the natural length of the one of the one or more conductive threads, a shape of the conductive thread between the two adjacent electrostatic protection sheets may be in a form of a wave pattern, a Z-shape, etc., which is not limited herein.

In some embodiments of the present disclosure, by setting the distance between the connection points of the two adjacent electrostatic protection sheets of the one of the one or more conductive threads connecting the two adjacent electrostatic protection sheets to be less than the natural length of the one of the one or more conductive threads, a length for stretching can be reserved for the conductive thread, which improves stretchability of the conductive thread, thereby improving stretchability of the device for monitoring the physiological signal.

In some embodiments, the conductive thread 443 is an elastic conductive thread. The conductive thread is elastically stretchable along an axial direction of the conductive thread (parallel to the length direction of the strap 410 in FIG. 4B). For example, when the strap 410 elongates, the conductive thread 443 also stretches in the axial direction. As another example, when the strap 410 rebounds from an elongated state, the conductive thread 443 also shortens in the axial direction. The conductive thread 443 may be a mixed thread of a metal thread (e.g., a silver thread) and an elastic thread (e.g., a rubber thread), etc.

In some embodiments of the present disclosure, by configuring the conductive thread as the elastic conductive thread, overall elasticity of the strap can be improved.

In some embodiments, the plurality of signal electrodes include a first signal electrode and a second signal electrode spaced apart along the length direction of the strap, the plurality of electrostatic protection sheets are electrically connected in sequence by one or more conductive threads (so that the plurality of electrostatic protection sheets are conductive with each other), and the plurality of electrostatic protection sheets form a conductive region that covers the first signal electrode and the second signal electrode in the length direction of the strap.

Exemplarily, as shown in FIG. 4A and FIG. 4B, the plurality of signal electrodes 420 include a first signal electrode 421 and a second signal electrode 422 spaced apart along the length direction of the strap. As shown in FIG. 4B, at least a portion of adjacent electrostatic protection sheets of the plurality of electrostatic protection sheets 440 are electrically connected via a conductive thread (e.g., the conductive thread 443), and the plurality of electrostatic protection sheets 440 form an integrated conductive region (a region where the plurality of electrostatic protection sheets 440 are located in FIG. 4B). To enable the plurality of electrostatic protection sheets 440 to form the integrated conductive region, a first portion (e.g., a plurality of electrostatic protection sheets on a left side) and a second portion (e.g., a plurality of electrostatic protection sheets on a right side) of the plurality of electrostatic protection sheets 440 are electrically connected with each other. That is, a last electrostatic protection sheet 446 of the first portion is electrically connected with a first electrostatic protection sheet 447 of the second portion (not shown in the figure).

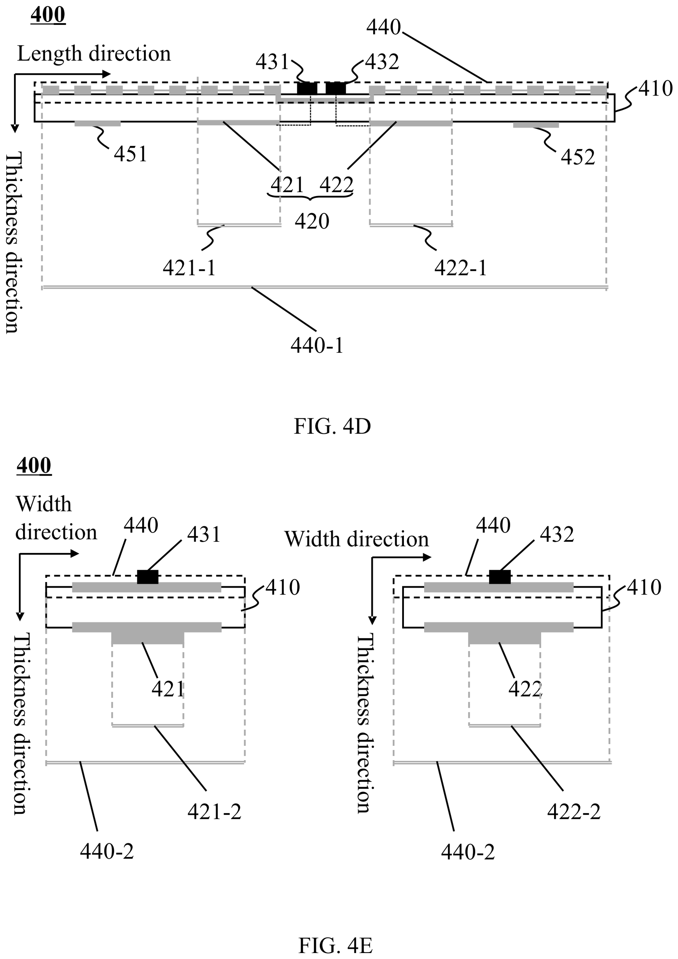

FIG. 4D is a schematic diagram illustrating a front projection view of another device for monitoring a physiological signal according to some embodiments of the present disclosure.

Exemplarily, as shown in FIG. 4D, a projection long side 421-1 of the first signal electrode 421 (a side of a projection region of the first signal electrode 421 that is parallel to the length direction of the strap 410) and a projection long side 422-1 of the second signal electrode 422 (a side of a projection region of the second signal electrode 422 that is parallel to the length direction of the strap 410) are both contained within a projection long side 440-1 of the conductive region formed by the plurality of electrostatic protection sheets 440 (a side of a projection region of the conductive region formed by the plurality of electrostatic protection sheets 440 that is parallel to the length direction of the strap 410). This arrangement causes the conductive region formed by the plurality of electrostatic protection sheets 440 to cover the first signal electrode 421 and the second signal electrode 422 in the length direction of the strap 410.

In some embodiments of the present disclosure, since static electricity that has a greatest effect on the signal electrode is the static electricity on the outer surface directly opposite the signal electrode and the static electricity near the outer surface, by arranging the conductive region to cover the two signal electrodes, the conductive region can disperse static electricity near the outer surface directly opposite the signal electrode, which can weaken or eliminate the effect of the static electricity on the signal electrode to the maximum extent.

In some embodiments, the conductive region covers the first signal electrode and the second signal electrode in a width direction of the strap.

FIG. 4E is a schematic diagram illustrating a side projection view of another device for monitoring a physiological signal according to some embodiments of the present disclosure. A left diagram and a right diagram in FIG. 4E are schematic diagrams illustrating side projection views observed from two opposite sides of the device 400 for monitoring the physiological signal monitoring, respectively.

Exemplarily, as shown in FIG. 4E, a projection wide side 421-2 of the first signal electrode 421 (a side of the projection region of the first signal electrode 421 that is parallel to the width direction of the strap 410) and a projection wide side 422-2 of the second signal electrode 422 (a side of the projection region of the second signal electrode 422 that is parallel to the width direction of the strap 410) are both contained within a projection wide side 440-2 of the conductive region formed by the plurality of electrostatic protection sheets 440 (a side of a projection region of the conductive region formed by the plurality of electrostatic protection sheets 440 that is parallel to the width direction of the strap 410). This arrangement causes the conductive region to cover the first signal electrode 421 and the second signal electrode 422 in the width direction of the strap 410.

In some embodiments of the present disclosure, by arranging the conductive region to cover the two signal electrodes in the width direction, the conductive region can disperse static electricity near the outer surface directly opposite the two signal electrodes, which can weaken or eliminate the effect of the static electricity on the signal electrodes to the maximum extent.

In some embodiments, the plurality of signal electrodes include a first signal electrode and a second signal electrode spaced apart along the length direction of the strap, a portion of the plurality of electrostatic protection sheets are electrically connected by a part of the one or more conductive threads to form a first conductive region that covers an outer side of the first signal electrode, another portion of the plurality of electrostatic protection sheets are electrically connected by another part of the one or more conductive threads to form a second conductive region that covers an outer side of the second signal electrode, and the first conductive region and the second conductive region are insulated from each other.

Exemplarily, as shown in FIG. 4B, at least a portion of adjacent electrostatic protection sheets of the first portion (e.g., a plurality of electrostatic protection sheets on the left side) of the plurality of electrostatic protection sheets 440 are electrically connected in sequence by conductive threads (e.g., the conductive thread 443) to form a first conductive region 444. The first conductive region 444 covers an outer side of the first signal electrode 421. At least a portion of adjacent electrostatic protection sheets of a second portion (e.g., a plurality of electrostatic protection sheets on the right side) of the plurality of electrostatic protection sheets 440 are electrically connected in sequence by the conductive threads to form a second conductive region 445. The second conductive region 445 covers an outer side of the second signal electrode 422. The first conductive region 444 and the second conductive region 445 are insulated from each other. That is, a last electrostatic protection sheet 446 of the first portion is not conductive with a first electrostatic protection sheet 447 of the second portion.

In some embodiments of the present disclosure, by arranging different conductive regions to cover corresponding electrodes, the different conductive regions can disperse static electricity near the outer surface directly opposite the electrodes, which can weaken or eliminate the effect of the static electricity on the electrodes to the maximum extent.

In some embodiments, the first conductive region covers the first signal electrode in the length direction of the strap, and the second conductive region covers the second signal electrode in the length direction of the strap.

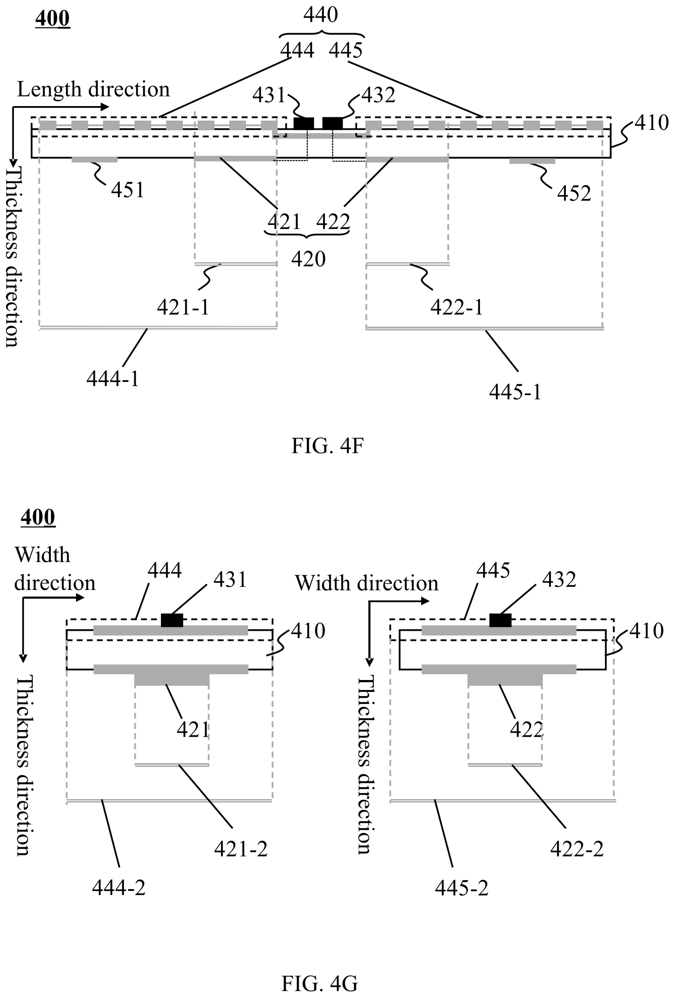

FIG. 4F is a schematic diagram illustrating another front projection view of another device for monitoring a physiological signal according to some embodiments of the present disclosure.

Exemplarily, as shown in FIG. 4F, the projection long side 421-1 of the first signal electrode 421 is contained within a projection long side 444-1 of the first conductive region 444 (a side of a projection region of the first conductive region 444 that is parallel to the length direction of the strap 410). The projection long side 422-1 of the second signal electrode 422 is contained within a projection long side 445-1 of the second conductive region 445 (a side of a projection region of the second conductive region 445 that is parallel to the length direction of the strap 410). This arrangement causes the first conductive region 444 to cover the first signal electrode 421 in the length direction of the strap 310, and the second conductive region 445 to cover the second electrode 422 in the length direction of the strap 310.

In some embodiments of the present disclosure, by arranging different conductive regions to cover corresponding electrodes in the length direction of the strap, the different conductive regions can disperse static electricity near the outer surface directly opposite the electrodes, which can weaken or eliminate the effect of the static electricity on the electrodes to the maximum extent.

In some embodiments, the first conductive region covers the first signal electrode in a width direction of the strap, and the second conductive region covers the second signal electrode in the width direction of the strap.

FIG. 4G is a schematic diagram illustrating another side projection view of another device for monitoring a physiological signal according to some embodiments of the present disclosure. A left diagram and a right diagram in FIG. 4G are schematic diagrams illustrating side projection views observed from two opposite sides of the device 400 for monitoring the physiological signal, respectively.

Exemplarily, as shown in FIG. 4G, the projection wide side 421-2 of the first signal electrode 421 is contained within a projection wide side 444-2 of the first conductive region 444 (a side of the projection region of the first conductive region 444 that is parallel to the width direction of the strap 410). The projection wide side 422-2 of the second electrode 422 is contained within a projection wide side 445-2 of the second conductive region 445 (a side of the projection region of the second conductive region 445 that is parallel to the width direction of the strap 410). This arrangement causes the first conductive region 444 to cover the first signal electrode 421 in the width direction of the strap 410, and the second conductive region 445 to cover the second signal electrode 422 in the width direction of the strap 410.

In some embodiments of the present disclosure, by arranging different conductive regions to cover corresponding electrodes in the width direction of the strap, the different conductive regions can disperse static electricity near the outer surface directly opposite the electrodes, which can weaken or eliminate the effect of the static electricity on the electrodes to the maximum extent.

The ground electrode is located on the inner surface of the strap and is configured to electrically connect the electrostatic protection sheets to the body of the user to provide the electrostatic shielding for the plurality of signal electrodes. A material of the ground electrode may be the conductive material with low hardness, e.g., the conductive rubber, the conductive silicone, etc. Exemplarily, as shown in FIG. 4A and FIG. 4B, a ground electrode 450 of the device 400 for monitoring the physiological signal may include a ground electrode 451 and a ground electrode 452, and the ground electrode 451 and the ground electrode 452 are disposed on the inner surface of the strap 410. When the user (e.g., the monitoring object 140) wears the device 400 for monitoring the physiological signal, the ground electrode 450 may attach to the skin of the user. The ground electrode 450 may be electrically connected with the plurality of electrostatic protection sheets 440 (not shown in the figure) to receive static electric charges on the outer surface received by the plurality of electrostatic protection sheets 440 and conduct the static electric charges into the human body to achieve the electrostatic shielding.

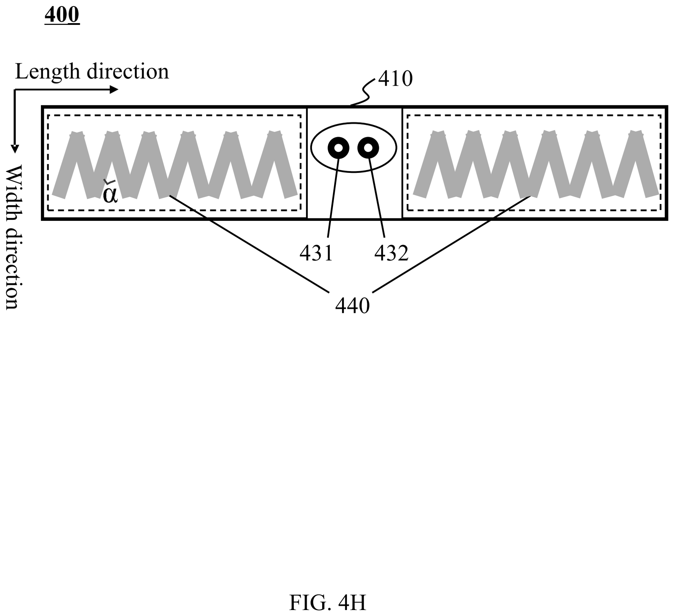

Different from a connection manner shown in FIG. 4B where the adjacent electrostatic protection sheets (e.g., the electrostatic protection sheet 441 and the electrostatic protection sheet 442) are connected to each other by one or more conductive threads, in other embodiments, the adjacent electrostatic protection sheets may also be directly connected with each other. FIG. 4H is an exemplary diagram illustrating a connection method of electrostatic protection sheets according to some embodiments of the present disclosure. As shown in FIG. 4H, the plurality of electrostatic protection sheets in each set are connected end-to-end in a stacked manner to form a bending structure. The adjacent electrostatic protection sheets are connected through end portions. Connection positions of the end portions are electrically connected to achieve conduction between the adjacent electrostatic protection sheets. In some embodiments, the bending structure formed by stacking the plurality of electrostatic protection sheets 440 is elastically stretchable in the length direction of the strap. Specifically, when an external force acts to stretch the strap 410 in the length direction, a bending angle α of the bending structure increases, a size of the bending structure in the length direction of the strap 410 also increases (i.e., the bending structure gradually changes from a bent shape to a linear shape). After the external force is removed, the bending structure gradually returns to its original state, thereby enabling the bending structure to be elastically stretchable in the length direction of the strap. The bending angle α refers to an included angle formed when end portions of two adjacent electrostatic protection sheets are connected. In some embodiments, a plurality of bending angles formed by the plurality of electrostatic protection sheets may be the same or different.

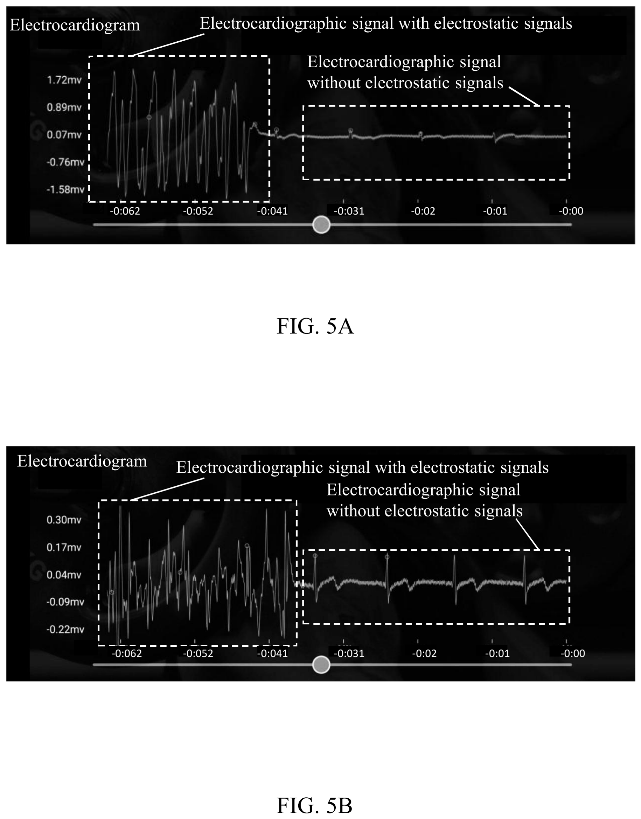

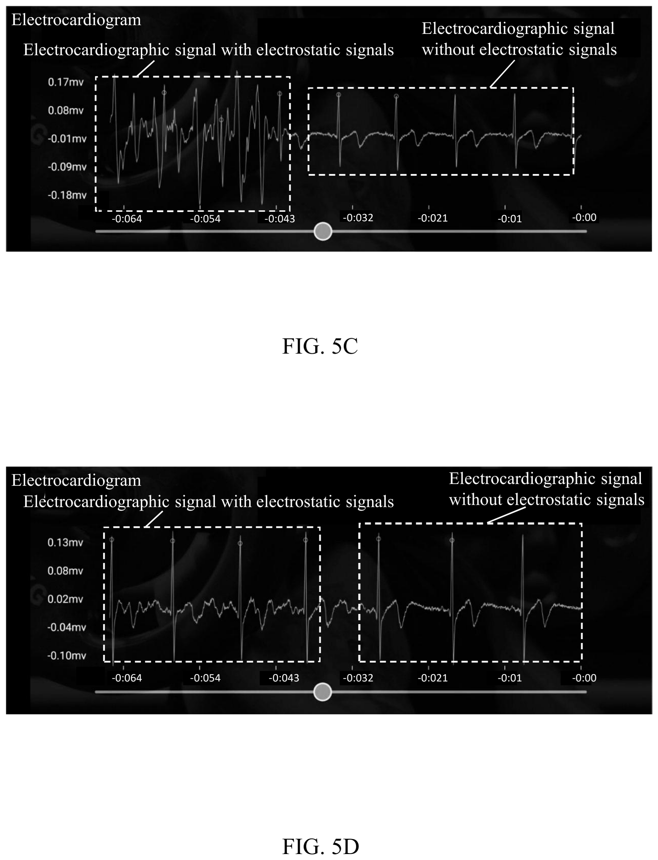

FIG. 5A is a diagram illustrating a verification result of a device for monitoring a physiological signal without an anti-static design in a high-impedance state according to some embodiments of the present disclosure; FIG. 5B is a diagram illustrating a verification result of a device for monitoring a physiological signal with an anti-static design in a high-impedance state according to some embodiments of the present disclosure; FIG. 5C is a diagram illustrating a verification result of a device for monitoring a physiological signal without an anti-static design in a low-impedance state according to some embodiments of the present disclosure; and FIG. 5D is a diagram illustrating a verification result of a device for monitoring a physiological signal with an anti-static design in a low-impedance state according to some embodiments of the present disclosure.

It is known that the hygroscopicity and water storage capacity of the material of the electrode (e.g., a signal electrode) of the device for monitoring the physiological signal may affect an anti-static effect. For example, compared to a material with weaker water absorption (e.g., polyester fiber), a material with stronger water absorption (e.g., cotton) has a stronger anti-static effect capability. This is because the presence of moisture may provide the conductive channel to help charge flow, thereby reducing electrostatic accumulation.

Precisely because the severity of the static electricity is strongly correlated with humidity, and a direct indicator reflecting humidity is the different impedance from the electrode to the human body. Therefore, the present disclosure verifies the anti-static effect of the device for monitoring the physiological signal in both high-impedance state and low-impedance state. The high-impedance state refers to that, when the device for monitoring the physiological signal is worn, an impedance between the first electrode and the second electrode (e.g., the first electrode 321 and the second electrode 322 shown in FIG. 3A) ranges from 1 MΩ to 10 MΩ. The low-impedance state refers to that, when the device for monitoring the physiological signal is worn, the impedance between the first electrode and the second electrode range ranges from 100 Ω to 500 kΩ.

An electrostatic triggering manner during verification is as follows: manually pull a garment made of cotton and polyester fiber blend in the width direction of the strap to cause friction between the garment and the strap, with a pulling amplitude and a frequency kept substantially consistent.

The high-impedance state and the low-impedance state may be achieved by applying water to the strap.

An impedance measurement manner is as follows: the monitoring object (e.g., the monitoring object 140) wears the device for monitoring the physiological signal, and then an impedance on the two connection ports (e.g., the connection port 323 and the connection port 324 shown in FIG. 3B) is measured. The impedance on the two connection ports is a sum of an impedance from one connection port to one electrode (with a very low impedance within 10 Ω), an impedance from the electrode to the human body, an impedance inside the human body (within 100 Ω), an impedance from the human body to another electrode, and an impedance from the another electrode to another connection port.

In FIG. 5A, FIG. 5B, FIG. 5C, and FIG. 5D, the electrocardiographic signals show almost no change, all around 0.13 mV. It should be noted that the intensities of the electrocardiographic signals in the aforementioned four figures may appear different, but they are actually substantially the same. This is because the scale of the vertical axis is different in each figure.

Comparing FIG. 5A and FIG. 5B in the high-impedance state, the electrostatic signal in FIG. 5A is larger, resulting in a too small signal-to-noise ratio in FIG. 5A, and the excessively large electrostatic signal affects the judgment of the electrocardiographic signal (as shown in FIG. 5A, the electrostatic signal almost submerges the electrocardiographic signal). In FIG. 5B, with the anti-static design, the electrostatic signal is significantly reduced, thereby improving the signal-to-noise ratio in FIG. 5B. The electrostatic signal does not affect the judgment of the electrocardiographic signal, indicating a good anti-static effect.

Similarly, comparing FIG. 5C and FIG. 5D in the low-impedance state, the signal-to-noise ratio of the device for monitoring the physiological signal with the anti-static design in FIG. 5D is also improved compared to the device for monitoring the physiological signal without the anti-static design in FIG. 5C.

It should be noted that the improvement in the signal-to-noise ratio is not very obvious in the low-impedance state compared to the high-impedance state. This is because the device has a stronger anti-static capability in the low-impedance state.

The basic concepts have been described above. Obviously, to those skilled in the art, the above detailed disclosure is merely an example and does not constitute a limitation to the present disclosure. Although not explicitly stated herein, those skilled in the art may make various modifications, improvements, and amendments to the present disclosure. Such modifications, improvements, and amendments are suggested in the present disclosure. Therefore, such modifications, improvements, and amendments still fall within the spirit and scope of the exemplary embodiments of the present disclosure.

Meanwhile, the present disclosure uses specific words to describe the embodiments of the present disclosure. Terms such as "one embodiment," "an embodiment," and/or "some embodiments" mean a certain feature, structure, or characteristic related to at least one embodiment of the present disclosure. Therefore, it should be emphasized and noted that mentioning "an embodiment" or "one embodiment" or "an alternative embodiment" two or more times in different locations in the present disclosure does not necessarily refer to the same embodiment. Furthermore, certain features, structures, or characteristics in one or more embodiments of the present disclosure may be appropriately combined.

Furthermore, unless explicitly stated in the claims, the order of processing elements and sequences, the use of numbers and letters, or the use of other names in the present disclosure are not intended to limit the order of the processes and methods of the present disclosure. Although the above disclosure discusses some currently considered useful inventive embodiments through various examples, it should be understood that such details are for illustrative purposes only. The appended claims are not limited to the disclosed embodiments. Instead, the claims are intended to cover all modifications and equivalent combinations that conform to the essence and scope of the embodiments of the present disclosure. For example, although the implementation of various components described above may be embodied in a hardware device, it may also be implemented as a software only solution, e.g., an installation on an existing server or mobile device.

Similarly, it should be noted that, in order to simplify the expression disclosed in the present disclosure and thereby facilitate the understanding of one or more inventive embodiments, various features are sometimes grouped into one embodiment, drawing, or description thereof in the foregoing description of the embodiments of the present disclosure. However, this disclosure method does not mean that the object of the present disclosure requires more features than those mentioned in the claims. Rather, claimed subject matter may lie in less than all features of a single foregoing disclosed embodiment.

In some embodiments, numbers describing the quantity of components or attributes are used. It should be understood that such numbers used in the description of the embodiments are modified by the modifiers "approximately," "about," or "substantially" in some examples. Unless otherwise stated, "approximately," "about," or "substantially" indicates that the number allows a variation of ±20%. Accordingly, in some embodiments, the numerical parameters used in the specification and claims are approximate values. These approximate values may vary according to the characteristics required by individual embodiments. In some embodiments, numerical parameters should consider the specified number of significant digits and adopt the method of general digit retention. Although the numerical ranges and parameters used to confirm the breadth of the scope in some embodiments of the present disclosure are approximate values, these numerical values are set as precisely as possible within the feasible range in specific embodiments.

For each patent, patent application, patent application publication, and other material, such as articles, books, specifications, publications, documents, etc., cited in the present disclosure, the entire content thereof is hereby incorporated by reference into the present disclosure. Application history documents that are inconsistent with or conflict with the content of the present disclosure are excluded. Documents that limit the broadest scope of the claims of the present disclosure (currently or subsequently attached to the present disclosure) are also excluded. It should be noted that if the description, definition, and/or use of terms in the ancillary materials of the present disclosure are inconsistent with or conflict with the description, definition, and/or use of terms in the present disclosure, the description, definition, and/or use of terms in the present disclosure shall prevail.

Finally, it should be understood that the embodiments described in the present disclosure are only used to illustrate the principles of the embodiments of the present disclosure. Other variations may also fall within the scope of the present disclosure. Therefore, by way of example and not limitation, alternative configurations of the embodiments of the present disclosure may be considered consistent with the teachings of the present disclosure. Accordingly, the embodiments of the present disclosure are not limited to the embodiments explicitly introduced and described in the present disclosure.

## Claims

1. A device for monitoring a physiological signal, comprising:
a strap configured to be worn on a body of a user, the strap including an inner surface and an outer surface; and
a plurality of electrodes arranged on the inner surface of the strap and configured to receive electrical signals from the body of the user, wherein one or more conductive threads are arranged on the strap, a first portion of the one or more conductive threads is located on the outer surface or between the inner surface and the outer surface, such that each of the plurality of electrodes is at least partially located between the first portion of the one or more conductive threads and the body of the user, and a second portion of the one or more conductive threads extends to the inner surface and electrically connects the first portion of the one or more conductive threads to the body of the user to provide electrostatic shielding for the plurality of electrodes.

2. The device of claim 1, wherein the strap is at least formed by weaving elastic threads, and the one or more conductive threads are interwoven through the inner surface and the outer surface of the strap.

3. The device of claim 1, wherein the strap is at least formed by a mixed weaving of elastic threads and the one or more conductive threads.

4. The device of claim 1, wherein at least a portion of the strap is formed by weaving the one or more conductive threads, and the one or more conductive threads penetrate through the inner surface and the outer surface in a thickness direction of the strap.

5. The device of claim 1, wherein the first portion and the second portion of the one or more conductive threads include a plurality of conductive channels arranged side by side in a width direction of the strap, respectively.

6. The device of claim 5, wherein each of the plurality of conductive channels is formed by weaving the one or more conductive threads in a wave pattern along a length direction of the strap.

7. The device of claim 1, wherein the plurality of electrodes include a first electrode and a second electrode spaced apart along a length direction of the strap, the first portion of the one or more conductive threads forms a first conductive region and a second conductive region insulated from each other, the first conductive region covers an outer side of the first electrode and is insulated from the first electrode, and the second conductive region covers an outer side of the second electrode and is insulated from the second electrode.

8. The device of claim 7, wherein the first conductive region covers the first electrode in the length direction of the strap, and the second conductive region covers the second electrode in the length direction of the strap.

9. The device of claim 7 or 8, wherein the first conductive region covers the first electrode in a width direction of the strap, and the second conductive region covers the second electrode in the width direction of the strap.

10. The device of claim 1, wherein the plurality of electrodes include a first electrode and a second electrode spaced apart along a length direction of the strap, the first portion of the one or more conductive threads forms a conductive region, and the conductive region covers the first electrode and the second electrode in the length direction.

11. The device of claim 10, wherein the conductive region covers the first electrode and the second electrode in a width direction of the strap.

12. The device of claim 1, wherein the plurality of electrodes are attached to the strap via one or more insulating layers, and the one or more insulating layers provide insulation between the plurality of electrodes and the one or more conductive threads.

13. The device of claim 1, wherein the one or more conductive threads include metal fibers, carbon fibers, compound fibers, or polymer fibers.

14. A device for monitoring a physiological signal, comprising:
a strap configured to be worn on a body of the user, the strap including an inner surface and an outer surface;
a plurality of signal electrodes arranged on the inner surface of the strap and configured to receive electrical signals from the body of the user;
two sets of electrostatic protection sheets, wherein one set of the two sets of electrostatic protection sheets is disposed on a first region of the outer surface of the strap or is disposed between the inner surface and the first region of the outer surface, the other set of the two sets of electrostatic protection sheets is disposed on a second region of the outer surface of the strap or is disposed between the inner surface and the second region of the outer surface, such that the plurality of signal electrodes are located between the two sets of electrostatic protection sheets and the body of the user, wherein each set of electrostatic protection sheets includes a plurality of electrostatic protection sheets; and
a ground electrode located on the inner surface of the strap and configured to electrically connect the two sets of electrostatic protection sheets to the body of the user to provide electrostatic shielding for the plurality of signal electrodes.

15. The device of claim 14, wherein at least a portion of the plurality of electrostatic protection sheets are electrically connected by one or more conductive threads.

16. The device of claim 15, wherein a distance between connection points of two adjacent electrostatic protection sheets and one of the one or more conductive threads connecting the two adjacent electrostatic protection sheets is less than a natural length of the one of the one or more conductive threads.

17. The device of claim 15, wherein the one or more conductive threads are elastic conductive threads, and the one or more conductive threads are elastically stretchable along an axial direction of the one or more conductive threads.

18. The device of claim 15, wherein the plurality of signal electrodes include a first signal electrode and a second signal electrode spaced apart along a length direction of the strap, the plurality of electrostatic protection sheets are electrically connected in sequence by the one or more conductive threads, and the plurality of electrostatic protection sheets form a conductive region that covers the first signal electrode and the second signal electrode in the length direction of the strap.

19. The device of claim 18, wherein the conductive region covers the first signal electrode and the second signal electrode in a width direction of the strap.

20. The device of claim 15, wherein the plurality of signal electrodes include a first signal electrode and a second signal electrode spaced apart along a length direction of the strap, a portion of the plurality of electrostatic protection sheets are electrically connected by a part of the one or more conductive threads to form a first conductive region that covers an outer side of the first signal electrode, another portion of the plurality of electrostatic protection sheets are electrically connected by another part of the one or more conductive threads to form a second conductive region that covers an outer side of the second signal electrode, and the first conductive region and the second conductive region are insulated from each other.

21. The device of claim 20, wherein the first conductive region covers the first signal electrode in the length direction of the strap, and the second conductive region covers the second signal electrode in the length direction of the strap.

22. The device of claim 20 or 21, wherein the first conductive region covers the first signal electrode in a width direction of the strap, and the second conductive region covers the second signal electrode in the width direction of the strap.

23. The device of claim 14, wherein the plurality of electrostatic protection sheets are arranged side by side along a length direction of the strap.

24. The device of claim 14, wherein the electrostatic protection sheets in each set are connected end-to-end in a stacked manner to form a bending structure, and the bending structure is elastically stretchable along a length direction of the strap.
